# EUROPEAN PATENT SPECIFICATION

(11) **EP 3 655 778 B1**
(45) Date of publication and mention of the grant of the patent: **07.06.2023**
(21) Application number: 18749741.7
(22) Date of filing: 20.07.2018
(51) Int. Cl.: G01N 33/574

(54) **AGRIN AS A MARKER FOR ENDOMETRIAL CANCER**
AGRIN ALS MARKER FÜR ENDOMETRIUMKARZINOM
AGRIN EN TANT QUE MARQUEUR DU CANCER ENDOMETRIAL

(30) Priority: 21.07.2017 EP 17382483
(43) Date of publication of application: 27.05.2020
(60) Divisional application: 23151625.3
(73) Proprietor: Fundació Hospital Universitari Vall d'Hebron - Institut de Recerca, 08035 Barcelona (ES)
(72) Inventor: MARTINEZ GARCÍA, Elena, 09002 Burgos (ES); COLÁS ORTEGA, Eva, 08020 Barcelona (ES); GIL MORENO, Antonio, 08172 Sant Cugat Del Vallès (ES); REVENTÓS PUIGJANER, Jaume, 08348 Cabrils (ES); DOMON, Bruno, CH-2534 Orvin (CH); LESUR, Antoine, L-8010 Strassen (LU); CAMPOY MONCAYO, Irene, 08028 Barcelona (ES)
(74) Representative: Hoffmann Eitle
(86) International application number: PCT/EP2018/069841
(87) International publication number: WO 2019/016400

(56) References cited:
- WO-A1-2017/190896
- SAYAN CHAKRABORTY ET AL: "An oncogenic role of Agrin in regulating focal adhesion integrity in hepatocellular carcinoma", NATURE COMMUNICATIONS, vol. 6, 29 January 2015 (2015-01-29), page 6184, XP055239818, DOI: 10.1038/ncomms7184
- ELENA MARTINEZ-GARCIA ET AL: "Development of a sequential workflow based on LC-PRM for the verification of endometrial cancer protein biomarkers in uterine aspirate samples & supplementary figures", ONCOTARGET, vol. 7, no. 33, 16 July 2016 (2016-07-16), pages 53102-53115, XP055503751,
- ALBA MOTA ET AL: "Genetic analysis of uterine aspirates improves the diagnostic value and captures the intra-tumor heterogeneity of endometrial cancers", MODERN PATHOLOGY, vol. 30, no. 1, 2 September 2016 (2016-09-02), pages 134-145, XP055503647, GB ISSN: 0893-3952, DOI: 10.1038/modpathol.2016.143
- S CABRERA ET AL: "Targeted proteomics identifies proteomic signatures in liquid-biopsies of the endometrium to diagnose endometrial cancer and assist in the prediction of the optimal surgical treatment", INTERNATIONAL JOURNAL OF GYNECOLOGICAL CANCER, vol. 27, no. supplement 4, 1 November 2017 (2017-11-01), pages 340-340, XP055503787, US ISSN: 1525-1438
- SAYAN CHAKRABORTY ET AL: "An oncogenic role of Agrin in regulating focal adhesion integrity in hepatocellular carcinoma", NATURE COMMUNICATIONS, vol. 6, 29 January 2015 (2015-01-29), page 6184, XP055239818, DOI: 10.1038/ncomms7184

## Description

### Background Art

Endometrial cancer (EC) is the most frequently observed invasive tumor of the female genital tract and the fourth most common cancer in women in developed countries, accounting for 61380 diagnosed cases and 10,920 estimated deaths in 2017 in the United States. Nowadays, 70% of the EC cases are diagnosed at early stages of the disease where the tumor is still localized within the endometrium and is associated with an overall 5-year survival rate of 96%. However, 30% of EC patients are diagnosed only at an advanced stage of the disease associated with a drastic decrease in the 5-year survival rate, which is reduced to 67% when myometrial invasion and/or lymph node affectation is already present and to 18% in cases of distant metastasis. Improving early diagnosis is hence a major issue to appropriately manage EC and decrease mortality associated with the disease.

Early detection of EC patients is favored by the presence of symptoms like abnormal vaginal bleeding present in 93% of women diagnosed with EC. However, many other benign disorders generate similar symptoms. Discrimination of patients with benign endometrial pathologies and with EC is only achieved after a tedious diagnostic process consisting of a pelvic examination and transvaginal ultrasonography followed by a confirmatory histopathological examination of an endometrial biopsy. The preferable biopsy used in this procedure is named uterine aspirate and/or pipelle biopsy and is obtained by a minimally invasive aspiration of endometrial fluid from inside the uterine cavity. Because the current diagnostic procedures on uterine aspirates rely on the presence of cellular material, this process has unfortunately a diagnostic failure and an associated inadequate sampling rate of 8% and 15%, respectively. This is increased in postmenopausal women up to 12% and 22%. In those cases, a biopsy guided by hysteroscopy needs to be performed, where this invasive technique presents an increased risk of complications, including uterine perforation, hemorrhage and possible harm to other organs.

To date, many studies have been conducted to identify EC protein biomarkers, mainly in tissue and serum samples (see for example DeSouza LV, et al, "Endometrial cancer biomarker discovery and verification using differentially tagged clinical samples with multidimensional liquid chromatography and tandem mass spectrometry", Mol Cell Proteomics MCP- 2007, vol. no.6, pp.:1170-8, or Kemik P, et al. "Diagnostic and prognostic values of preoperative serum levels of YKL-40, HE-4 and DKK-3 in endometrial cancer", Gynecol Oncol- 2016; vol. no.140, pp.:64-9). None of them have been translated into clinical utility.

Some documents have correlated the genetic analysis and mutations in particular genes with the diagnostic of endometrial cancer and the prognostic for grade identification of the disease. One example is the document of Mota et al., "Genetic analysis of uterine aspirates improves the diagnostic value and captures the intra-tumor heterogeneity of endometrial cancers", Modern Pathology-2016, vol. 30, no. 1, pp.: 134-145. This document shows that intra-tumor genetic heterogeneity may be represented in uterine aspirates, providing the advantage of being more representative of the entire tumor than the data from samples of a single region of the tumor. Some of the revealed informative mutations in genes were identified in CTNNB1 gene, PIK3 or PTEN gene.

Although the biopsies should provide information about tumor histology and tumor grade to help in the risk stratification of the EC patients and guide the surgical staging procedure, unfortunately, the limited number of cells available for examination and the high inter-observer variability in the pathological interpretation results in 40-50% of discordances in EC histotype and grade between biopsies and final hysterectomy specimens. Therefore, the identification of sensitive, specific, and reproducible biomarkers that improve diagnosis, prognosis and preoperative assessment of the histological type and grade of EC tumors is crucial to appropriately manage EC patients and decrease mortality and morbidity associated with this disease.

Among the suptypes or manifestations variety of EC, endometroid endometrial cancer (EEC) is the most common histology in EC and has a good prognosis when compared with non-endometrioid EC cases (NEEC). NEEC represents about 20% of all EC cases but accounts for more than 50% of recurrences and deaths from EC. Among NEEC, the serous EC (SEC) is the most common subtype. There are no availabe tests for discriminating between these two histologies with different outcomes.

Concluding, in spite of the efforts made, there is still the need of biomarkers allowing the diagnosis, and even the prognosis of endometrial cancer with high sensitivity and specificity, in particular the need of biomarkers for clinical practice.

### Summary of Invention

Inventors have determined that certain protein markers detectable in exosomes isolated from uterine fluid, give valuable diagnostic information in endometrial cancer (EC). Many of these proteins were validated in uterine fluid itself without isolation of the exosome fraction. Hence, the proteins are meaningful diagnostic biomarkers allowing discrimination with high sensitivity and specificity between EC and non-cancer controls when analyzed in the uterine fluid.

In addition, inventors have determined that some proteins that can be detected in said uterine fluid, and also in the exosome fraction of said uterine fluid, are meaningful prognostic biomarkers of endometrial cancer (EC). These proteins allow discrimination between endometrial cancer subtypes with high sensitivity and high specificity, and thus they allow minimizing the risk of false positive and false negative classification among these subtypes.

Moreover, being the proteins detectable in samples that are obtained without invasive practices, such as in the uterine fluid samples, which in turn are routine sample types in the EC diagnostics, supposes a real advantage and improvement in the clinical practice of EC patient classification and managing.

In previous studies, inventors demonstrated that the fluid fraction of uterine aspirates have an important value for the screening of EC protein biomarkers (see for example Martinez-Garcia E, et al. "Development of a sequential workflow based on LC-PRM for the verification of endometrial cancer protein biomarkers in uterine aspirate samples", Oncotarget -2016, vol. no. 7(33), pp.:53102-53114). This document shows a study carried out with a sequential workflow based on LC-PRM, illustrating the importance of a biomarker verification phase to fill the gap between discovery and clinical practice. This study highlighted the benefit of 26 proteins to diagnose EC patients from non-EC patients (controls).

One aspect of the invention is a method of diagnosing endometrial cancer, the method comprising determining the level of expression of AGRIN in an uterine fluid sample from the female genital tract.

In a particular embodiment of the method, the uterine fluid sample is uterine aspirate fluid sample from the female genital tract.

In another particular embodiment of the method according to the invention, the uterine fluid sample is exosome-containing fraction isolated from a uterine aspirate.

In another particular embodiment, the method further comprises determining the level of expression of one or more proteins selected from PERM, OSTP, CTNB1, CAYP1, XPO2, NGAL, SG2A1, CADH1, SPIT1, MMP9, NAMPT, LDHA, CASP3, PRDX1, MIF, K2C8, CAPG, MUC1, ANXA1, HSPB1, PIGR, CH10, CD44, CLIC1, TPIS, GSTP1, GTR1, ENOA, PDIA1, KPYM, ANXA2 and FABP5.

In another particular embodiment of the method when the uterine fluid sample is the exosome-containing fraction, it comprises determining in the exosome-containing fraction isolated from a uterine aspirate the level of expression of at least one set of proteins selected from the group consisting of: AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; and AGRIN, BCAM.

In a particular embodiment of the method, in which the uterine fluid sample is uterine aspirate fluid sample, it comprises determining in the uterine aspirate the level of expression of at least one set of proteins selected from the group consisting of: AGRIN, MMP9, PODXL; and AGRIN, MMP9.

In a particular embodiment of the method of diagnosing endometrial cancer, it comprises determining the level of expression of at least one set of proteins selected from the group consisting of: AGRIN, CD81, TERA; AGRIN, CD59, MVP; AGR2, AGRIN, CD81; AGRIN, CD166, MVP; AGRIN, CD81; AGRIN, CD166; AGRIN, CD59; and AGRIN, MMP9, in an exosome-containing fraction isolated from UA; and determining the level of expression of at least one set of proteins selected from the group consisting of: MMP9, PODXL, RAB8A; MMP9, PODXL, RSSA; AGRIN, MMP9, PODXL; MMP9, PODXL, VAMP8; MMP9, MX1; MMP9, RSSA; MMP9, MVP; MMP9, RAB8A; MMP9, VAMP8; BCAM, MMP9; MMP9, AGRIN in a uterine aspirate.

In another particular embodiment of the method, the level of expression is determined at the protein level.

In yet another particular embodiment of the method of diagnosing endometrial cancer, the protein level is determined by an assay or technology selected from the group consisting of an immunoassay, a bioluminescence assay, a fluorescence assay, a chemiluminescence assay, electrochemistry assay, mass spectrometry, and combinations thereof.

The method of the invention is carried out, in a particular embodiment, by means of a kit comprising a solid support and means for detecting the level of expression of AGRIN.

There are kits and tools comprising antibodies for determining AGRIN in isolated samples of cancer patients. See for example the document of Chakraborty et al., "An oncogenic role of Agrin in regulating focal adhesion integrity in hepatocellular carcinoma. Nature Communications-2015, vol. 6, page 6184. In this document the detection of Agrin, a protein involved in the oncogenic signalling, was detected by means of antibodies that provoked the reduction of the said oncogenic signalling and tumour growth.

Another aspect of the invention is, nonetheless, a new and particular kit comprising a solid support and means for detecting the level of expression of AGRIN, and means for detecting the level of expression of the proteins of at least one set of proteins selected from the group consisting of AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; AGRIN, BCAM; AGRIN, MMP9, PODXL; AGRIN, MMP9; and AGRIN, MMP9, PIGR, wherein the means are antibodies or a fragments thereof able to bind to the target protein(s).

In a particular embodiment of the kit of the invention, the antibodies or fragments thereof able to bind to the target protein(s) for detecting the level of expression of the proteins are antibodies or fragments thereof able to bind to the target protein(s) for carrying out an assay or technology selected from the group consisting of an immunoassay, a bioluminescence assay, a fluorescence assay, a chemiluminescence assay, electrochemistry assay, and combinations thereof.

More in particular, the kits comprise as the means for detecting the level of expression of the proteins, antibodies or fragments thereof.

In another particular embodiment of the kits, they are kits for carrying out an enzyme-linked immunosorbent assay.

In another particular embodiment, the kits further comprise a pannel diagram, to categorize an individual sample.

In another particular embodiment of the method of diagnosing according to the invention, it comprises:
a) determining, *in vitro,* the level of expression of AGRIN, in an uterine fluid sample from the female;
b) optionally determining, *in vitro,* the level of expression of AGRIN, in an exosome-containing fraction isolated from uterine fluid; and
c) comparing the level of step (a) and optionally that of step (b) with a reference control level, wherein if the level determined in step (a) and optionally that of step (b) is higher than the reference control level, it is indicative that the subject is suspicious of suffering endometrial carcinoma.

Yet another aspect of the invention is a method of deciding or recommending whether to initiate a medical regimen of a subject suspicious of suffering endometrial carcinoma, which method comprises the steps of:
a) determining, *in vitro,* the level of expression of AGRIN in an uterine fluid sample from the female;
b) optionally determining, *in vitro,* the level of expression of AGRIN in an exosome-containing fraction isolated from uterine fluid; and
c) comparing the level of step (a) and optionally that of step (b) with a reference control level, wherein if the level determined in step (a) and optionally that of step (b) is higher than the reference control level, it is indicative that the subject is suspicious of suffering endometrial carcinoma
   wherein:
   i) if the subject is diagnosed of suffering from endometrial carcinoma, or of being suspicious of suffering from endometrial carcinoma, then the initiation of the medical regimen is recommended, and
   ii) if the patient is diagnosed of not suffering from endometrial carcinoma, the follow-up is performed optionally in consideration of the result of an examination of the patient by a physician.

By determining the marker level in a test sample, the skilled person can establish, additionally, which is the most suitable therapy that can be recommended, because the level detected in the sample may reflect the extension (i.e., severity) of the disease.

The invention includes a method as defined in any of the previous aspects, in which after the determination of the level of expression of AGRIN, and the proteins of the sets of proteins including AGRIN and listed above for the diagnosis of EC, the following computer-implemented steps are carried out: said level(s) are given a value and/or a score, and optionally are computed in a mathematical formula to obtain a computed value; wherein in function of the said level(s), score(s) and or computed value(s), a decision is taken between the options of suffering or not from EC.

Another aspect of the invention is the use of a kit comprising a solid support and means for detecting the level of expression of AGRIN and, optionally, means for detecting the level of expression of the proteins of at least one set of proteins selected from the group consisting of AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; AGRIN, BCAM; AGRIN, MMP9, PODXL; AGRIN, MMP9; and AGRIN, MMP9, PIGR, for the diagnosis of endometrial cancer, wherein the means are antibodies or a fragments thereof able to bind to the target protein(s).

In a particular embodiment of the said use of a kit, the kit is one wherein the antibodies or fragments thereof able to bind to the target protein(s) for detecting the level of expression of the proteins are antibodies or fragments thereof able to bind to the target protein(s) for carrying out an assay or technology selected from the group consisting of an immunoassay, a bioluminescence assay, a fluorescence assay, a chemiluminescence assay, electrochemistry assay, and combinations thereof.

The methods of the invention give important diagnostic and prognostic information, since accurate prognosis can be done because two different hystological manifestations of EC, namely endometrioid endometrial cancer (EEC) and non-endometroid endometrial cancer (NEEC), can be differentiated, being EEC of better likely outcome (i.e. better prognosis). In other words, the determination of the proteins in the uterine fluid sample allows differentially diagnosing EEC and NEEC.

As will be depicted in the examples below, AGRIN allowed the diagnose of EC or to accurately prognose EC, in terms that it was differentially expressed in isolated samples from patients suffering from EEC and from patients suffering from NEEC. Diagnostic value could be differentially detected due to differential expression in uterine fluid samples of EC subjects in relation with non-EC samples (healthy controls). Prognostic value of AGRIN results in that it was significantly increased in EEC subtype in comparison with the levels in NEEC subtype. So that, it allowed classification of tumors of the most prevalent histological subtypes. The protein is thus a usable tool for improving diagnosis, prognosis and/or preoperative risk assessment, and for assisting in the prediction of the optimal surgical treatment. Also as indicated below, some protein panels (protein combinations) with this protein have been developed that allow for the accurate discrimination of EC vs non-EC samples and also for accurate discrimination EC histological types (AUC of 0.99). In combination with the histopathological examination, these panels are expected to preclude the need of more invasive diagnostic samplings and help to predict the optimal surgical treatment for EC patients.

Importantly, the protein biomarker AGRIN object of the present invention can be assessed by easy and low cost methods, such as immunochemistry, chemoluminiscent assay, or ELISA, platforms which are widely available in hospitals. Consequently, this protein biomarker can be easily implemented as routine clinical diagnostic and/or prognostic kits with reduced costs for the health system. In addition, a diagnostic kit test based on the biomarker provided by the present invention can ameliorate the current process of diagnosis and prognosis, conferring to uterine aspirates the ability of providing valuable diagnostic and prognostic information of the disease.

According to aspects and embodiments of the invention, diagnosis and prognosis of EC can be performed using a mathematical algorithm that assesses a detectable level of biomolecules, proteins, antibodies, and/or mRNA, comprising one or more of the biomarkers of diagnosis and prognosis of EC described above, either in conjunction with or independent of other clinical parameters, to correctly categorize an individual sample as originating from a healthy patient, a patient with a non-malignant disease of the endometrium, a patient with a pre-malignant disease of the endometrium, a patient with endometrial cancer, or, as described above, to further categorize an individual sample as originating from a subject with a specific histological subtype of endometrial cancer, a subject having a specific histological grade or stage of the disease, or a subject with a specific molecular subtype of EC. In the sense of the invention, the term "algorithm" is also synonymous of pannel or decision diagrams, predictors and combinatory of data to correctly categorize an individual sample.

The classification algorithm may be as simple as determining whether or not the amount of AGRIN is above or below a particular cut-off number. When multiple biomarkers inlcuding AGRIN of the sets listed above are used, the classification algorithm may be a linear regression formula. Alternatively, the classification algorithm may be the product of any of a number of learning algorithms. In the case of complex classification algorithms, it may be necessary to perform the algorithm on the data, thereby determining the classification, using a computer, e.g., a programmable digital computer. In either case, one can then record the status on tangible medium, for example, in computer-readable format such as a memory drive or disk or simply printed on paper. The result also could be reported on a computer screen. This algorithm is used as diagnostic and/or prognostic method, and is in particular part of the kits for carrying out the methods disclosed in former aspects.

After the determination of the level of expression of AGRIN for the diagnosis and/or for the prognosis of EC in the function of the said level, score and/ or computed value, a decision is taken between the options of suffering or not from pre-malignant lesions, and/or EC, and/or between the options of suffering among different EC subtypes.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The present invention provides new biomarkers for the diagnosis and for prognosis of endometrial cancer in the female genital tract fluid.

The term "diagnosis" is known to the person skilled in the art. As used herein "diagnosis" is understood as becoming aware of a particular medical condition complication or risk in a subject; the determination of the nature of the disease or condition; or the distinguishing of one disease or condition from another. It refers both to the process of attempting to determine or identify the possible disease or disorder, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification. In general terms, diagnostic markers listed in this description are those protein differentially detected at level expression in isolated samples of controls (non-cancer individuals) versus endometrial cancer samples (including several types of EC).

The *in vitro* method of the first aspect of the invention can be performed with a sample of: (a) an asymptomatic subject, (b) a subject which has already been identified as being suspicious of suffering from endometrial cancer, (c) a subject already diagnosed of endometrial cancer, as complementary confirmation diagnostic assay or (d) a subject with high risk of suffering the disease.

In the present invention, the term "reference control level" referred to in the methods of the any of the aspects of the invention is to be understood as a predefined value of a given molecular marker or a combination of the given molecular markers, in the present case any of the proteins listed in the first or second aspects as well as in particular embodiments, which is derived from the levels of said molecular marker or markers in a sample or group of samples. If the level of expression is determined at the protein level, then the "reference expression level" is a predefined value of protein quantity, whereas if the level of expression is determined at the mRNA level, then the "reference expression level" is a predefined value of mRNA quantity. The samples are taken from a subject or group of subjects wherein the presence, absence, stage, histological subtype or grade, or course of the disease has been properly performed previously. This value is used as a threshold to discriminate subjects wherein the condition to be analyzed is present from those wherein such condition is absent (i.e. subject having endometrial cancer from subjects free of endometrial cancer), to determine the histological subtype of the disease, the risk of developing or of being suffering from endometrial carcinoma, among others. This reference control level is also useful for determining whether the subject has to initiate a medical regimen and how effective the regimen is. The subject or subjects from whom the "reference control level" is derived may include subject/s wherein the condition is absent, subject/s wherein the condition is present, or both. The skilled person in the art, making use of the general knowledge, is able to choose the subject or group of subjects more adequate for obtaining the reference control level for each of the methods of the present invention. Methods for obtaining the reference value from the group of subjects selected are well-known in the state of the art (Burtis C. A. et al., 2008, Chapter 14, section "Statistical Treatment of Reference Values") In a particular case "reference control level" is a cut-off value defined by means of a conventional ROC analysis (Receiver Operating Characteristic analysis). As the skill person will appreciate, optimal cut-off value will be defined according to the particular applications of the diagnostic or prognostic method: purpose, target population for the diagnosis or prognosis, balance between specificity and sensibility, etc.

"Prognosis" as used herein refers to the prediction of the probable progression and outcome of a disease. It includes: neoplasm grading (attempt to express in replicable terms the level of cell differentiation in neoplasms as increasing anaplasia correlates with the aggressiveness of the neoplasm), neoplasm staging (attempt to express in replicable terms the extent of the neoplasm in the patient), neoplasm histological subtype, and neoplasm molecular subtype. As used herein prognosis means, in particular embodiments, differentiation between endometrioid endometrial cancer and non-endometrioid endometrial cancers.

The term "fluid sample from the female genital tract" refers to a fluid produced by the uterine organ forming part of the female genital tract and which has been taken by aspiration, such as vacuum aspiration (i.e., "uterine aspirate sample"), or by a cornier pipelle, and/or any other method that retrieves fluid from the uterine cavity. Thus, it includes uterine washings. According to the present invention, the aspiration of the fluid is in particular performed without a previous step of saline infusion. That is, the term "aspirate" does not encompass those samples resulting from uterine washings.

In the present invention, "Exosomes" interchangeably referred as "Extracellular vesicles", "microvesicles", "exosome-like vesicles" or, "uterosomes" are cell-derived vesicles that are present in many eukaryotic fluids, including blood, urine, and cultured medium of cell cultures. The reported diameter of exosomes is between 30 and 100 nm, which is larger than low-density lipoproteins (LDL) but much smaller than, for example, red blood cells. Exosomes are either released from the cell when multivesicular bodies fuse with the plasma membrane or released directly from the plasma membrane. Exosomes can potentially be used for diagnosis, for prognosis, for therapy, and as biomarkers for health and disease. For "exosome-containing fraction isolated from UA" is to be understood any purified fraction from the uterine aspirate that comprises mainly exosomes. Non-limiting examples of methods for isolating exosomes from uterine aspirates are detailed below.

In another embodiment, the method of diagnosis of EC comprises determining the level of expression of AGRIN in a exosome-containing fraction isolated from UA; and the level of expression of AGRIN in an uterine fluid sample from the female genital tract, this later sample without isolation or purification of exosomes.

In another particular embodiment, the method of diagnosis of EC further comprises determining the level of expression of one or more proteins selected from the group consisting of: PERM, OSTP, CTNB1, CAYP1, XPO2,NGAL, SG2A1, CADH1, SPIT1, MMP9, NAMPT, LDHA, CASP3, PRDX1, MIF, K2C8, CAPG, MUC1, ANXA1, HSPB1, PIGR, CH10, CD44, CLIC1, TPIS, GSTP1, GTR1, ENOA, PDIA1, KPYM, ANXA2 and FABP5.

As indicated, in another particular embodiment, optionally in combination with any embodiment above or below, the method of diagnosis of EC comprises determining the level of expression of at least one set of proteins (i.e. biomarkers) selected from the group consisting of: AGRIN, CD81, TERA; AGRIN, CD59, MVP; AGR2, AGRIN, CD81; AGRIN, CD166, MVP; AGRIN, CD81; AGRIN, CD166; AGRIN, CD59; and AGRIN, MMP9, in an exosome-containing fraction isolated from UA; and determining the level of expression of at least one set of proteins (i.e. biomarkers) selected from the group consisting of: MMP9, PODXL, RAB8A; MMP9, PODXL, RSSA; AGRIN, MMP9, PODXL; MMP9, PODXL, VAMP8; MMP9, MX1; MMP9, RSSA; MMP9, MVP; MMP9, RAB8A; MMP9, VAMP8; BCAM, MMP9; MMP9, AGRIN in a uterine aspirate.

Another provision of the invention is the use of AGRIN as *in vitro* marker for diagnosing EC in an uterine fluid sample from the female genital tract. This provision can also be formulated as an *in vitro* method for detecting one or more endometrial cancer markers in a subject, comprising: (a) obtaining a fluid sample from the female genital tract; and (b) detecting in the sample an amount of at least one endometrial cancer marker selected from AGRIN in an uterine fluid sample from the female genital tract, said marker giving information of the diagnosis of EC. In general terms, it is encompassed the use of AGRIN as *in vitro* marker for diagnosing endometrial cancer in an isolated uterine fluid sample from the female genital tract.

Another aspect of the invention is the use of kits, comprising a solid support and antibodies or fragments thereof able to bind to the target protein for detecting the level of expression of AGRIN , for the diagnosis of EC.

In a particular embodiment of the use of said kits, they further comprise means for detecting the level of expression of the proteins of at least one set of proteins selected from the group consisting of AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; AGRIN, BCAM; AGRIN, MMP9, PODXL; AGRIN, MMP9; and AGRIN, MMP9, PIGR, for the diagnosis of endometrial cancer, wherein the means are antibodies or fragments thereof able to bind to the target protein(s).

In another particular embodiment of the use of the kits, the antibodies or fragments thereof able to bind to the target protein(s) for detecting the level of expression of the proteins are antibodies or fragments thereof able to bind to the target protein(s) for carrying out an assay or technology selected from the group consisting of an immunoassay, a bioluminescence assay, a fluorescence assay, a chemiluminescence assay, electrochemistry assay, and combinations thereof.

Uniprot database accession numbers of all the herewith listed proteins correspond to versions accessible on July 07, 2017.

AGRIN has the Uniprot database accession number O00468. This protein is a heparin sulfate basal lamina glycoprotein involved in the formation and the maintenance of the neuromuscular junction.

MVP, also known as major vault protein, has the Uniprot database accession number Q14764. It is involved in signal transduction.

VAMP8, also known as vesicle-associated membrane protein 8, has the Uniprot database accession number Q9BV40. It is a soluble N-ethylmaleimide-sensitive factor-attachment protein receptor (SNARE) involved in autophagy through the direct control of autophagosome membrane fusion with the lysososome membrane.

RAB8A, also known as Ras-related protein Rab-8A, has the Uniprot database accession number P61006. It is a small GTPases Rab involved in intracellular membrane trafficking.

MX1, also known as interferon-induced GTP-binding protein Mx1, has the Uniprot database accession number P20591. It has antiviral activity against a wide range of RNA viruses and some DNA viruses.

IMB1, also known as importin subunit beta-1, has the Uniprot database accession number Q14974. It is involved in nuclear protein import.

TERA, also known as transitional endoplasmic reticulum ATPase, has the Uniprot database accession number P55072. This protein is involved in the formation of the transitional endoplasmic reticulum.

BCAM, also known as basal cell adhesion molecule, has the Uniprot database accession number P50895. This protein is a Laminin alpha-5 receptor.

ANXA2, also known as Annexin A2, has the Uniprot database accession number P07355. This protein is a Calcium-regulated membrane-binding protein which inhibits PSCK9-enhanced LDLR degradation.

RSSA, also known as 40S ribosomal protein SA or Laminin receptor 1, has the Uniprot database accession number P08865. It is required for the assembly and/or stability of the 40S ribosomal subunit.

AGR2, also known as anterior gradient protein 2 homolog or HPC8, has the Uniprot database accession number O95994. This protein is involved in MUC2 post-transcriptional synthesis and secretion.

PODXL, also known as Podocalyxin or GCTM-2 antigen, has the Uniprot database accession number O00592. It is involved in the regulation of adhesion, cell morphology and cancer progression.

CD59, also known as CD59 glycoprotein or MAC-inhibitory protein, has the Uniprot database accession number P13987. It is involved in the inhibition of the complement membrane attack complex (MAC) action.

CD166, also known as CD166 antigen, has the Uniprot database accession number Q13740. It is involved in both heterotypic and homotypic cell-cell contacts.

CD81, also known as CD81 antigen or Tetraspanin-28, has the Uniprot database accession number P60033. This protein may be involved the regulation of lymphoma cell growth.

PIGR, also known as polymeric immunoglobulin receptor, has the Uniprot database accession number P01833, June 26, 2007 - v4. This receptor binds polymeric IgA and IgM at the basolateral surface of epithelial cells.

CTNB1, also known as catenin beta-1, has the Uniprot database accession number P35222, February 1, 1994 - v1. It acts as a negative regulator of centrosome cohesion and blocks anoikis of malignant kidney and intestinal epithelial cells.

CAYP1, also known as calcyphosin, has the Uniprot database accession number Q13938, November 1, 1997 - v1. It is a calcium-binding protein that may play a role in cellular signaling events.

CADH1, also known as cadherin-1 or E-cadherin, has the Uniprot database accession number P12830, July 1, 1993 - v3. This protein is involved in mechanisms regulating cell-cell adhesions, mobility and proliferation of epithelial cells. Has a potent invasive suppressor role.

CD44, also known as CD44 antigen, has the Uniprot database accession number P16070, October 5, 2010 - v3. Mediates cell-cell and cell-matrix interactions through its affinity for HA, and possibly also through its affinity for other ligands such as osteopontin, collagens, and matrix metalloproteinases (MMPs).

XPO2, also known as exportin-2, has the Uniprot database accession number P55060, March 29, 2005 - v3. Among others, this protein has been disclosed as exporting receptor for importin-alpha, mediating importin-alpha re-export from the nucleus to the cytoplasm after import substrates (cargos) and binding cooperatively to importin-alpha and to the GTPase Ran in its active GTP-bound form.

SG2A1, also known as mammaglobin-B, has the Uniprot database accession number O75556, November 1, 1998 - v1. It may bind androgens and other steroids.

ENOA, also known as alpha-enolase, has the Uniprot database accession number

P06733, January 23, 2007 - v2. It is a multifunctional enzyme that, as well as its role in glycolysis, plays a part in various processes such as growth control, hypoxia tolerance and allergic responses. May also function in the intravascular and pericellular fibrinolytic system due to its ability to serve as a receptor and activator of plasminogen on the cell surface of several cell-types such as leukocytes and neurons. Stimulates immunoglobulin production.

CAPG, also known as macrophage-capping protein, has the Uniprot database accession number P40121, November 30, 2010 - v2. It is a calcium-sensitive protein which reversibly blocks the barbed ends of actin filaments but does not sever preformed actin filaments. It may play an important role in macrophage function.

PRDX1, also known as peroxiredoxin-1, has the Uniprot database accession number Q06830, June 1, 1994 - v1. It is involved in redox regulation of the cell.

CLIC1, also known as Chloride intracellular channel protein 1, has the Uniprot database accession number O00299, January 23, 2007 - v4. It insert into membranes and form chloride ion channels. Channel activity depends on the pH. Membrane insertion seems to be redox-regulated and may occur only under oxydizing conditions. Involved in regulation of the cell cycle.

PDIA1, also known as protein disulfide-isomerase, has the Uniprot database accession number P07237, November 1, 1997 - v3. It catalyzes the formation, breakage and rearrangement of disulfide bonds.

KPYM, also known as pyruvate kinase PKM, has the Uniprot database accession number P14618, January 23, 2007 - v4. It is a glycolytic enzyme that catalyzes the transfer of a phosphoryl group from phosphoenolpyruvate (PEP) to ADP, generating ATP and plays a general role in caspase independent cell death of tumor cells.

GSTP1, also known as glutathione S-transferase P, has the Uniprot database accession number P09211, January 23, 2007 - v2. It regulates negatively CDK5 activity via p25/p35 translocation to prevent neurodegeneration

GTR1 has the Uniprot database accession number P11166, October 3, 2006 - v2. It is a facilitative glucose transporter. This isoform may be responsible for constitutive or basal glucose uptake. It has a very broad substrate specificity; can transport a wide range of aldoses including both pentoses and hexoses.

CH10, also known as 10 kDa heat shock protein, mitochondrial, has the Uniprot database accession number P61604, January 23, 2007 - v2. It is essential for mitochondrial protein biogenesis, together with CPN60. Binds to CPN60 in the presence of Mg-ATP and suppresses the ATPase activity of the latter.

MIF, also known as macrophage migration inhibitory factor, has the Uniprot database accession number P14174, January 23, 2007 - v4. It is involved in the innate immune response to bacterial pathogens.

TPIS, also known as triosephosphate isomerase, has the Uniprot database accession number P60174, October 19, 2011 - v3. This protein is involved in the pathway gluconeogenesis, which is part of carbohydrate biosynthesis.

NGAL, also known as Neutrophil gelatinase-associated lipocalin, has the Uniprot database accession number P80188, November 1, 1995 - v2. It is involved in apoptosis due to interleukin-3 (IL3) deprivation and in innate immunity.

LDHA, also known as L-lactate dehydrogenase A chain, has the Uniprot database accession number P00338, January 23, 2007 - v2. This protein is involved in step 1 of the subpathway that synthesizes (S)-lactate from pyruvate.

Some of these proteins have been related with EC gradation in tissue samples. However, no meaningful data are correlated with values in uterine fluid samples of the female genital tract. As above exposed, detection of markers in uterine fluids (aspirates or washings) imply the advantage of collecting data from routine sampling in clinical practice of the gynecological diseases, avoiding costs and importantly, tissue biopsies.

As indicated, in another particular embodiment of the method, the level of expression is determined at the protein level.

In a more particular embodiment, the protein level is determined by an assay or technology selected from the group consisting of an immunoassay, a bioluminescence assay, a fluorescence assay, a chemiluminescence assay, electrochemistry assay, mass spectrometry, and combinations thereof.

In another particular embodiment, the level of expression of protein is determined using an antibody or a fragment thereof able to bind to the protein.

More in particular, said antibody or fragment o thereof forms part of a kit.

The invention also relates to a computer-implemented method for carrying out the method of differential diagnosis as disclosed above and as defined in any of the embodiments, in which after the determination of the level of expression of one or more of the proteins for the diagnosis and/or for the prognosis of EC, said level(s) are given a value and/or a score, and optionally are computed in a mathematical formula to obtain a computed value; wherein in function of the said level(s), score(s) and or computed value(s), a decision is taken between the options of suffering or not from EC and/or between the options of suffering among different EC subtypes.

Particular combination AGRIN, MMP9, PIGR was determined of high prognostic value when the levels of expression of the proteins were detected in a uterine aspirate. Next Table B shows AUC and Confidence interval (CI) of 95 % values. Data derive from the analysis of samples from EEC and NEEC. In Table C, also AUC values are indicated.

On the other hand, Table A shows AUC values and CI of 95 % of the following combination: AGRIN, BCAM, giving important prognostic information when the levels of expression of the proteins were detected in an exosome-containing fraction isolated from uterine aspirate. Data derive from the analysis of samples from EEC and NEEC.

**TABLE A**

| **PROTEIN 1** | **PROTEIN 2** | **AUC** | **CI 95% inf.** | **CI 95% sup.** | **Sample** |
|---|---|---|---|---|---|
| AGRIN | BCAM | 0,903 | 0,821 | 0,984 | Exosomes |

**TABLE B**

| **PROTEIN 1** | **PROTEIN 2** | **PROTEIN 3** | **AUC** | **CI 95% inf.** | **CI 95% sup.** | **Sample** |
|---|---|---|---|---|---|---|
| AGRIN | MMP9 | PIGR | 0,98 | 0,946 | 1 | Uterine aspirate |

In any of the embodiments provided above or below, for any of the aspects of the invention, the level of expression is determined at the protein level. In this embodiment, the protein marker(s) include, but do not limit to, native-sequence peptides, isoforms, chimeric polypeptides, all homologs, fragments, and precursors of the markers, including modified forms of the polypeptides and derivatives thereof. In a more particular embodiment the protein level is determined by an assay or technology selected from the group consisting of an immunoassay, a bioluminescence assay, a fluorescence assay, a chemiluminescence assay, electrochemistry assay, mass spectrometry, and combinations thereof.

In particular embodiments provided above or below, the level of expression is determined by immunochemistry.

The term "immunochemistry" as used herein refers to a variety of techniques for detecting antigens (usually proteins and peptides, and in the present case any of the proteins listed above alone or in combination) in a sample by exploiting the principle of antibodies binding specifically to said antigens. Visualizing an antibody-antigen interaction can be accomplished in a number of ways. In the most common instance, an antibody is conjugated to an enzyme, such as peroxidase, that can catalyse a colour-producing reaction. Alternatively, the antibody can also be tagged to a fluorophore, such as fluorescein or rhodamine. The immunochemistry technique can be direct or indirect. The direct method is a one-step staining method and involves a labeled antibody (e.g. FITC-conjugated antiserum) reacting directly with the antigen. While this technique utilizes only one antibody and therefore is simple and rapid, the sensitivity is lower due to little signal amplification, such as with indirect methods, and is less commonly used than indirect methods. The indirect method involves an unlabeled primary antibody (first layer) that binds to the target antigen in the sample and a labeled secondary antibody (second layer) that reacts with the primary antibody. This method is more sensitive than direct detection strategies because of signal amplification due to the binding of several secondary antibodies to each primary antibody if the secondary antibody is conjugated to the fluorescent or enzyme reporter.

Further amplification can be achieved if the secondary antibody is conjugated to several biotin molecules, which can recruit complexes of avidin-, streptavidin or Neutravidin-enzyme. The indirect method, aside from its greater sensitivity, also has the advantage that only a relatively small number of standard conjugated (labeled) secondary antibodies needs to be generated. With the direct method, it would be necessary to label each primary antibody for every antigen of interest. It must be borne in mind that immunochemistry techniques can also be used to detect certain nucleic acid sequences if a tagged nucleic acid probe (designed to specifically bind to a certain target nucleic acid sequence) can later on be detected with a labelled antibody. Thus, the detection of the protein could be performed by using a tagged nucleic acid designed to bind a specific sequence of the target protein RNA, and then detecting said tagged nucleic acid with a labelled antibody which selectively binds to the tag.

Immunoassay procedures suitable include enzyme-linked immunosorbent assays (ELISA, such as multiplex ELISA), enzyme immunodot assay, agglutination assay, antibody-antigen-antibody sandwich assay, antigen-antibody-antigen sandwich assay, immunochromatography, or other immunoassay formats well-known to the ordinarily skilled artisan, such as radioimmunoassay, as well as protein microarray formats.

In one embodiment, in combination with any of the embodiments provided above or below, the level of expression of protein is determined by an immunoassay.

In another embodiment, in combination with any of the embodiments provided above or below, the level of expression of protein is determined by ELISA; more in particular multiplex ELISA.

Alternatively, the level of expression of protein can be determined by bioluminescence, fluorescence, chemiluminescence, electrochemistry, or mass spectrometry.

Alternatively, the level of expression of protein can be determined by measuring the levels of proteotypic peptides of the protein (peptides with an amino acid sequence uniquely associated with the studied protein in a given proteome) by mass spectrometry.

In another embodiment, in combination with any of the embodiments provided above or below, the level of expression of protein is determined using an antibody or a fragment thereof able to bind to the target protein(s).

The term "antibody or a fragment thereof able to bind to the target protein(s)" is to be understood as any immunoglobulin or fragment thereof able to selectively bind the target protein. It includes monoclonal and polyclonal antibodies. The term "fragment thereof encompasses any part of an antibody having the size and conformation suitable to bind an epitope of the target protein. Suitable fragments include F(ab), F(ab') and Fv. An "epitope" is the part of the antigen being recognized by the immune system (B-cells, T-cells or antibodies).

The antibodies used for specific detection can be polyclonal or monoclonal. There are well known means in the state of the art for preparing and characterizing antibodies. Methods for generating polyclonal antibodies are well known in the prior art. Briefly, one prepares polyclonal antibodies by immunizing an animal with the protein; then, serum from the immunized animal is collected and the antibodies isolated. A wide range of animal species can be used for the production of the antiserum. Typically the animal used for production of antisera can be a rabbit, mouse, rat, hamster, guinea pig or goat.

Moreover, monoclonal antibodies (MAbs) can be prepared using well-known techniques. Typically, the procedure involves immunizing a suitable animal with the protein associated with the disease. The immunizing composition can be administered in an amount effective to stimulate antibody producing cells. Methods for preparing monoclonal antibodies are initiated generally following the same lines as the polyclonal antibody preparation. The immunogen is injected into animals as antigen. The antigen may be mixed with adjuvants such as complete or incomplete Freund's adjuvant. At intervals of two weeks, approximately, the immunization is repeated with the same antigen.

In another particular embodiment of the third aspect, the means to carry out the invention form part of a kit. The antibody or fragment thereof for detecting the target protein(s) can be included in a kit. The kit may additionally comprise means (additives, solvents) to visualize the antibody-protein interactions.

These antibodies can be used, as previously said, as "means" for determining the expression of the target proteins in the fifth aspect of the invention.

Thus, in a particular embodiment of the first and further method aspects of the invention, the level of expression of a protein is determined using an antibody or a fragment thereof able to bind to the protein.

In another particular embodiment, said antibody or fragment thereof forms part of a kit.

Alternatively, the level of expression is determined at the mRNA level.

In one embodiment, the amount of mRNA of each one of the markers are detected via polymerase chain reaction using, for example, oligonucleotide primers that hybridize to one or more polynucleotide endometrial cancer markers or complements of such polynucleotides. Within other embodiments, the amount of mRNA is detected using a hybridization technique, employing oligonucleotide probes that hybridize to one or more polynucleotide endometrial cancer markers or complements of such polynucleotides.

When using mRNA detection, the method may be carried out by combining isolated mRNA with reagents to convert to cDNA according to standard methods well known in the art, treating the converted cDNA with amplification reaction reagents (such as cDNA PCR reaction reagents) in a container along with an appropriate mixture of nucleic acid primers; reacting the contents of the container to produce amplification products; and analyzing the amplification products to detect the presence of one or more of the polynucleotide endometrial cancer markers in the sample. For mRNA, the analyzing step may be accomplished using Northern Blot analysis to detect the presence of polynucleotide endometrial cancer markers in the sample. The analysis step may be further accomplished by quantitatively detecting the presence of polynucleotide endometrial cancer markers in the amplification product, and comparing the quantity of marker

detected against a panel of expected values for the known presence or absence of such markers in normal and malignant tissue derived using similar primers.

In another embodiment, the invention provides a method wherein mRNA is detected by: (a) isolating mRNA from a sample and combining the mRNA with reagents to convert it to cDNA; (b) treating the converted cDNA with amplification reaction reagents and nucleic acid primers that hybridize to one or more of the polynucleotide endometrial cancer markers endometrial cancer marker to produce amplification products; (c) analyzing the amplification products for determining the amount of mRNA present encoding the protein endometrial cancer marker; and (d) comparing the determined amount of mRNA to an amount detected against a panel of expected values for normal and diseased tissue (e.g. , malignant tissue) derived using similar methods.

In particular embodiments of the invention, RT-PCR can be used to amplify the mRNA for protein endometrial cancer markers for detection and analysis. Other embodiments of the invention use quantitative RT-PCR to quantitatively determine amount of mRNA for protein endometrial cancer markers. Further embodiments of the invention use real time RT-PCR for quantification and analysis.

Regarding the device or kits of the invention, said kits are, in particular embodiments of the invention provided for the analysis of patient samples Devices of interest include arrays, where the reagents are spatially separated on a substrate such as a slide, gel, multi-well plate, etc. Alternatively, the reagents may be provided as a kit comprising reagents in a suspension or suspendable form, e.g. reagents bound to beads. Reagents of interest include reagents specific for autoantibody markers. Such reagents may include antigenic proteins or peptides, and the like. Such devices or kits may further comprise cytokine-specific antibodies or fragments thereof; and the like.

Particular combination of next Table E was determined of high diagnostic value when the levels of expression of the proteins were detected in a uterine aspirate. Next Table E shows AUC and Confidence interval (CI) of 95 % values. Data derive from the analysis of samples from non-EC and EC (including EEC and NEEC).

**TABLE E. Combination of diagnostic value in uterine aspirate**

| **PROTEIN 1** | **PROTEIN 2** | **PROTEIN 3** | **AUC** | **CI 95% inf.** | **CI 95% sup.** | **Sample** |
|---|---|---|---|---|---|---|
| AGRIN | MMP9 | PODXL | 0,979 | 0,953 | 1 | Uterine aspirate |
| MMP9 | AGRIN | | 0,94 | 0,89 | 0,992 | Uterine aspirate |

**TABLE F. Combinations of diagnostic value in an exosome-containing fraction isolated from uterine aspirate**

| **PROTEIN 1** | **PROTEIN 2** | **PROTEIN 3** | **AUC** | **CI 95% inf.** | **CI 95% sup.** | **Sample** |
|---|---|---|---|---|---|---|
| AGRIN | CD81 | TERA | 0,944 | 0,902 | 0,986 | Exosomes |
| AGRIN | CD59 | MVP | 0,944 | 0,902 | 0,986 | Exosomes |
| AGR2 | AGRIN | CD81 | 0,943 | 0,903 | 0,982 | Exosomes |
| AGRIN | CD166 | MVP | 0,938 | 0,896 | 0,98 | Exosomes |
| AGRIN | CD81 | | 0,934 | 0,89 | 0,979 | Exosomes |
| AGRIN | CD166 | | 0,926 | 0,878 | 0,975 | Exosomes |
| AGRIN | CD59 | | 0,921 | 0,871 | 0,97 | Exosomes |
| AGRIN | MMP9 | | 0,9 | 0,848 | 0,961 | Exosomes |

On the other hand, Table F shows AUC values and CI of 95 % of the following combinations: AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9, giving important diagnostic information when the levels of expression of the proteins were detected in an exosome-containing fraction isolated from uterine aspirate. Data derive from the analysis of samples from non-EC and EC (including EEC and NEEC).

The "solid support" includes a nitrocellulose membrane, glass or a polymer. The most commonly used polymers being cellulose, polyacrylamide, nylon, polystyrene, polyvinyl chloride or polypropylene. The solid supports may be in the form of strips, tubes, beads, discs or microplates, or any other surface suitable for conducting an immunoassay.

Binding assays for measuring biomarker levels may use solid phase or homogenous formats. Suitable assay methods include sandwich or competitive binding assays. Examples of sandwich immunoassays are described in U.S. Pat. No. 4,168,146 and U.S. Pat. No. 4,366,241, both of which are incorporated herein by reference in their entireties. Examples of competitive immunoassays include those disclosed in U.S. Pat. No. 4,235,601, U.S. Pat. No. 4,442,204 and U.S. Pat. No. 5,208,535, each of which are incorporated herein by reference in their entireties.

Multiple biomarkers may be measured using a multiplexed assay format, e.g., multiplexing through the use of binding reagent arrays, multiplexing using spectral discrimination of labels, multiplexing of flow cytometric analysis of binding assays carried out on particles, e.g., using the Luminex^{®} system.

The assays of the present invention may be conducted by any suitable method. In one embodiment, biomarker levels are measured in a single sample, and those measurement may be conducted in a single assay chamber or assay device, including but not limited to a single well of an assay plate, a single assay cartridge, a single lateral flow device, a single assay tube, etc. Biomarker levels may be measured using any of a number of techniques available to the person of ordinary skill in the art, e.g., direct physical measurements (e.g., mass spectrometry) or binding assays (e.g., immunoassays, agglutination assays and immunochromatographic assays). The method may also comprise measuring a signal that results from a chemical reactions, e.g., a change in optical absorbance, a change in fluorescence, the generation of chemiluminescence or electrochemiluminescence, a change in reflectivity, refractive index or light scattering, the accumulation or release of detectable labels from the surface, the oxidation or reduction or redox species, an electrical current or potential, changes in magnetic fields, etc. Suitable detection techniques may detect binding events by measuring the participation of labeled binding reagents through the measurement of the labels via their photoluminescence (e.g., via measurement of fluorescence, time-resolved fluorescence, evanescent wave fluorescence, up-converting phosphors, multi-photon fluorescence, etc.), chemiluminescence, electrochemiluminescence, light scattering, optical absorbance, radioactivity, magnetic fields, enzymatic activity (e.g., by measuring enzyme activity through enzymatic reactions that cause changes in optical absorbance or fluorescence or cause the emission of chemiluminescence). Alternatively, detection techniques may be used that do not require the use of labels, e.g., techniques based on measuring mass (e.g., surface acoustic wave measurements), refractive index (e.g., surface plasmon resonance measurements), or the inherent luminescence of an analyte.

In another embodiment, the kit is a microarray including a defined set of genes encoding protein endometrial cancer markers. All the embodiments provided above for particular AGRIN protein to be analyzed, whose expression is significantly altered by endometrial disease, are also particular embodiments of microarrays.

The *in vitro* methods of the invention provide diagnostic and/or prognostic information. In one embodiment, the methods of the invention further comprise the steps of (i) collecting the diagnostic and/or prognostic information, and (ii) saving the information in a data carrier.

In the sense of the invention a "data carrier" is to be understood as any means that contain meaningful information data for the diagnosis and/or prognosis of endometrial carcinoma, such as paper. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the diagnosis/prognosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Example 1. Biomarkers of diagnosis and prognosis of EC in exosomes derived from uterine fluid, and in the uterine fluid.

### 1. Samples, reagents and methods

### 1.1 Patients and sample description

Patients were recruited at three different institutions: HUVH (Hospital Universitari Vall d'Hebron, Barcelona, Spain), HUAV (Hospital Universitari Arnau de Vilanova, Lleida, Spain) and UMCF (University Medical Center of Freiburg, Freiburg, Germany). Each participating institution obtained ethical approval and samples were obtained after the participants signed the informed consent.

Uterine aspirates (UAs) were obtained by aspiration with a Cornier Pipelle (Gynetics Medical Products). Samples were placed in 1.5 mL tubes and kept on ice through all the processing which included addition of phosphate buffered saline (PBS) in a 1:1 ratio (v/v), gently pipetting of the sample, and centrifugation at 2500g (4 °C) in a F45-30-11 rotor (Eppendorf Microcentrifuge 5417R) for 20 min to remove the cellular fraction. The remaining supernatant (SN) fraction, was then aliquoted and frozen at -80 °C until needed.

For exosome (abbreviated ELVs) isolation, ELVs were obtained from the SNs of UAs by differential centrifugation, following a modification of a previously described ELVs isolation protocol by Thery et al. http://www.ncbi.nlm.nih.gov/pubmed/18228490. Briefly, SNs were thawed and diluted in PBS to a final volume of 25 mL. A centrifugation step at 10,000xg (4 °C) for 30 min was performed on a Thermo Scientific Heraeus MultifugeX3R Centrifuge (FiberLite rotor F15-8x-50c) to remove cell debris, macroparticles and apoptotic bodies. The resulting pellet enriched in MVs was resuspended in 50 µL of PBS and frozen at -80 °C. Then, the supernatant was transferred to ultracentrifuge tubes (Beckman Coulter) and filled with PBS to perform a first ultracentrifugation step at 100,000xg (4 °C) for 2 h on a Thermo Scientific Sorvall WX UltraSeries Centrifuge with an AH-629 rotor. The supernatant of this second centrifugation was the soluble fraction and was frozen at -80 °C. This first pellet was resuspended in PBS and again centrifuged at 100,000xg (4 °C) for 1 h. The final pellet enriched in ELVs (possibly along with microvesicles (MVs) and some remaining apoptotic bodies) was resuspended in 50 µL of PBS. Five microliters from MVs and ELVs pellets were reserved at -80 °C for particle size distribution and quantification by NTA while the rest of the sample was frozen at -80 °C for protein extraction.

### 1.2 Protein extraction and identification by Liquid chromatography-parallel reaction monitoring (LC-PRM) analysis

For the discovery phase, ELVs were resuspended in a lysis buffer composed of 40 mM Tris pH 8, 300 mM NaCl, 10 mM EDTA, 2 %Triton X-100 and 1:100 protease inhibitors (Sigma-Aldrich) in a 1:1 ratio (v/v). Then, samples were frozen at -20°C for at least 8 hours and then thawed on ice and sonicated five cycles of 5 seconds at amplitude 100% (Labsonic M, Sartorius Stedim Biotech) to ensure membrane disruption. The extracted proteins were stored at -20°C until needed.

Validation phase was performed on ELVs and on the uterine aspirate (UA) without need of isolating ELVs. To extract proteins from ELVs for the validation phase, the detergent contained in the lysis buffer was changed for <1% NP-40, to make the protein extraction already suitable for direct in-solution digestion and LC-MS/MS. The rest of the procedure remained the same. To extract proteins from the fluid fractions of UAs; those were sonicated 5 cycles of 5 seconds each at 100% amplitude (Labsonic M, Sartorius Stedim Biotech) to disrupt microvesicles, protein aggregates, and/or mucus present in the sample. Then, the Albumin & IgG depletion spin trap Kit (GE Healthcare) was used following the manufacturer's instructions to remove albumin and immunoglobulin G from the samples. All the extracted proteins were stored at -20°C until needed. Diagnostic and prognostic value data were also obtained with uterine fluid sample (ELVs or UA without isolation of ELVs) in which no depletion of albumin and immunoglobulin G was carried out.

For the discovery phase, peptides dissolved in 0.1% formic acid were first loaded on a 150 µm ID x 20 cm nano-LC in a house packed column (Jupiter C18, 3 µm, 300 Å, Phenomenex, Torrance, CA) and separated with an EASY nanoLC system (Thermo Scientific). For the elution of the peptides, a 1 hour linear gradient of 5-40% ACN (0.2% FA) at a constant flow rate of 600nL/min was used. The LC system was coupled to a QExactive plus Orbitrap tandem mass spectrometer (Thermo Fisher Scientific) and RAW files were acquired with XCalibur software (Thermo Fisher Scientific). Tandem mass spectra were performed with a Top-12 method with precursor isolation window of m/z 2.0. The resolution was 70.000 at m/z 400 for the survey scan (with AGC 1e⁶, maximal injection time 200 ms and a scan range of m/z 300-2000) and 17.500 for MS/MS spectra (AGC at 5e⁵, maximal injection time 50 ms and scan range m/z 200-2000). Normalized collision energy (NCE) was set at 25 and exclusion time was set to 10 s.

Regarding protein identification, for SILAC quantification, RAW files were analyzed using MaxQuant (version 1.3.0.3). Default parameters were taken: MS/MS fragment error tolerance of 20 ppm, Carbamidomethylatin (C) fixed and Oxidation (M) as well as Acetyl (Protein N-term) as variable modification. Arginine (Arg10) and lysine (Lys8) were selected for the heavy label and applied the "match between run" option. The RAW files were searched against the HUMAN SwissProt database. For the Presence/Absence analysis, a label free analysis was done with PEAKS 7.0 (http://www.bioinfor.com/). For the database search, RAW files were searched against the Human Uniprot database containing 37254 entries. For the search parameters, precursor error tolerance was set at 10 ppm and for the MS2 fragments the tolerance was at 0.01Da. A maximum of 2 misscleavages for Trypsin were allowed. Carbamidometylation (C), Deamidation (NQ) and Oxidations (M) were variable modifications. Only peptides with false discovery rates (FDR) lower 1% were considered. For validation of potential biomarkers, PEAKS results were uploaded in Scaffold proteome software (http://www.proteomesoftware.com/).

The statistical analysis of the discovery phase was performed to obtain two different outpouts: (1) A qualitative data consisting of proteins that were present or absent in the different subgroups of patients; and (2) a quantitative data consisting of the expression measures obtained from SILAC ratios representing relative abundance of each protein vs. internal standards.

For the qualitative data, a Fisher exact test was applied to each protein to compare presence/absence between groups (p-value < 0.001). For the quantitative data, a Student t-test was performed between each pair of groups in order to select differentially expressed proteins. The test was performed only for those proteins that were present in more than 4 individuals per group. In order to address the problem of multiple comparisons derived from performing many tests (one per protein), the p-values were adjusted to obtain a strong control over FDR using the Benjamini and Hockberg methods (adj.pvalue < 0.25 and FC > 1.3).

All analyses were performed using the statistical program "R" (R version 3.2, Copyright (C) 2015 The R Foundation for Statistical Computing).

A total of 54 proteins were selected for the targeted experiment by selected reaction monitoring (SRM) based on the results from the discovery phase (49), together with 5 candidates selected from previous results of our group. Two unique peptides per protein were selected to be monitored by SRM based on their detectability in previous mass spectrometric experiments. For each selected peptide, an isotopically-labeled version (¹⁵N₂,¹³C₆-Lysine, 15N4,13C6-Arginine) was bought and spiked in each sample to be used as internal standard. Internal standards were spiked in a concentration within the linear response range, which was established for each individual peptide based on experimental dilution curves (data not shown). In parallel, isotopically-labeled peptides were mixed and used to generate MS2 spectral library and retention time knowledge database.

A total of 85 endogenous peptides and their corresponding internal standards were measured by SRM in Lys-C and trypsin-digested samples from an independent cohort of 107 patients. The five most intense transitions per peptide were monitored on a triple quadrupole mass spectrometer (5500 Q-Trap, AB Sciex Instruments, Foster, CA, USA) equipped a reversed-phase chromatography 25-cm column with an inner diameter of 75 µM, packed with 1.9 µM C18 particles (NikkyoTechnos Co., Ltd. Japan) and a 2-cm pre-column (Acclaim PepMap 100, C18, 15 µM, 100A). Loading buffer: H2O with 0.1% formic acid; eluting buffer: ACN, 0.1% formic acid. The flow rate used was 250 nL/min and a chromatographic gradient ranging from 5 to 40% eluting buffer in 40 min was used. Blank runs were performed between the SRM measurements of biological samples to avoid sample carryover. Measurements were done in scheduled SRM mode, using a window of 300 seconds and a target cycle time of 1.5 seconds.

For the data analysis of the verification phase, transition groups corresponding to the targeted peptides were evaluated with Skyline v2.5 based on the co-elution of the transition traces associated with a targeted peptide, both in its light and heavy form; the correlation between the light SRM relative intensities and the heavy counterpart. All peptide peaks were visually inspected. Areas of all transitions were used as input for the linear mixed-effects model implemented in the MSstats Skyline plug-in (v3.3) to calculate protein fold-changes and adjusted p-values among the different sample groups.

For the development of predictors, MSstats was used to estimate the quantity of proteins present in all samples based on log2-transformed transition areas, which were then used as input variables to a logistic regression model between defined groups. The classification ability of each protein was evaluated within 2/3 of the dataset using the area under the curve (AUC) as performance indicator. The most discriminative protein was selected as the first classifier. Most discriminative proteins were repeatedly added while increasing AUC values (deltaAUC > 0.02). The procedure of classification evaluation was repeated 500 times using a different subset of patients in each iteration. Sample subsets were generated by randomly selecting patients from the original cohort without replacement and balanced for each sample subgroup. A final consensus model was comprised of the combination of proteins, which were selected most in 500 repeats. The final model was fitted to the full dataset and the predictive accuracy was quantified using the area under the ROC curve, sensitivity, specificity, and accuracy. The pROC package for the statistical program "R" was used to draw ROCs, to calculate AUCs and other performance values (i.e. sensitivity, specificity, and accuracy); these were obtained considering the "optimal cutoff" as the point were the sum of sensitivities and specificities reached the maximum value.

### 2. Results

### 2.1. Discovery phase

For the discovery phase of this project, uterine aspirates from 10 EEC (also abbreviated EC1) patients, 10 from NEEC (also abbreviated EC2, and that were particularly SEC) and 10 Control patients (N=30) were used to isolate ELVs.

Once ensured the purity of the vesicles isolated, the same amount of Super-SILAC mix (internal standard) was spiked in each of the 30 samples (EEC n=10, NEEC n=10 and CTRL n=10) in a 2:1 *Heavy*/*Light* ratio, right before the separation by 1D-SDS-PAGE. Each gel lane was sliced into 10 bands, and each band was subjected to trypsin digestion to extract peptides, resulting in 300 individual LC-MS/MS runs. Protein database searching of MS/MS data resulted in the overall identification of 2138 proteins, considering only those proteins identified with at least 1 unique peptide, and those peptides with a false discovery rate (FDR) lower than 1%. The MS/MS spectra were further processed with the software MaxQuant leading to the quantification of 920 proteins, that is an overall quantification rate of a 43% of the identified proteome.

A total of 152 proteins were differentially expressed with adjusted p-value < 0,25 and fold-change lower than -1,3 or higher than 1,3. From those, 147 proteins were potential diagnostic biomarkers (differential from the comparison CTRL vs. EC), and 28 proteins were potential prognostic biomarkers (differential from the comparison EEC vs. NEEC). In parallel to the quantitative analysis, a *presence*/*absence* study was conducted in order to take into consideration proteins that failed to be quantified because they lacked the heavy counterpart but were still relevant for being present in a certain group. For this, RAW files were reanalyzed with the PEAKS software and a Fisher Test was performed to select those proteins significantly present in a group (p-value < 0.001). A total of 30 candidates were included following this analysis, corresponding to 29 potential diagnostic biomarkers and 9 potential prognostic biomarkers.

Altogether, a final list of 54 candidates was generated combining (i) the relative quantification analysis, (ii) the presence/absence study, and (iii) biological criteria such as the exclusion of proteins whose family was overrepresented in the candidate list, or proteins down-regulated in cancer; always respecting the statistical restrictions stated above. From those 54 candidate biomarkers, 50 corresponded to diagnostic biomarkers and 15 to prognostic biomarkers, from which 37 had only diagnostic potential, 2 had only prognostic potential and 13 had both, diagnostic and prognostic potential.

### 2.2. Verification of protein biomarkers in UAs ELVs

Once obtained the list of 54 candidates described in the previous section, it was aimed to verify the potential of those candidates by using LC-SRM in a new and bigger cohort of patients. As in the discovery phase study, the ELVs fraction of UAs was the selected sample of analysis. In addition to evaluate the individual potential of each marker to diagnose EC and differentiate between histological subtypes, in here there was also sought to generate diagnostic and prognostic models by combining different candidates. Moreover, it was assessed the classification power of each candidate in the whole fluid fraction of UAs in an independent cohort.

A total of 107 patients were recruited for the verification phase (cohort B), divided in three groups: EEC or EC1 (n=45), NEEC or EC2 (n=21) and CTRL (n=41). Specifically, NEEC corresponded to patients diagnosed with serous endometrial cancer (SEC). From those, ELVs from UAs were isolated and characterized as seen in previous sections (data not shown). The list of 54 protein candidate biomarkers generated in the discovery phase was verified by LC-SRM. To do so, two unique tryptic peptides were selected per protein, from which a total of 85 endogenous peptides were finally monitored by scheduled-SRM. However, there were only detected 69 of the 85 peptides corresponding to 51 proteins; three of the candidates (TNR6, CLH1 and PSB3) were not detected in any of the samples. As a result of this SRM experiment, there was observed that 43 out of the 48 (89.6%) potential diagnostic biomarkers (i.e. significant differences in abundance were observed between CTRL and EC) were also significant in this verification phase (adj.pvalue < 0.01), thus confirming their potential as individual diagnostic biomarkers. A total of 29 out of the 45 quantified proteins presented high accuracy to individually discriminate between EC and CTRL cases. The 5 most significant individual biomarkers were AGRIN (AUC= 0.90, CI95: 0.85-0.96), TACD2 (AUC= 0.87, CI95: 0.81-0.94), SORT (AUC=0.86, CI95: 0.79-0.93), MVP (AUC= 0.86, CI95: 0.78-0.93) and FAS (AUC= 0.85, CI95: 0.78-0.92). Table 2.1 below shows the list of selected biomarkers for EC diagnosis in ELVs.

Regarding the potential prognostic biomarkers (i.e. comparison of EC1 (EEC) vs. EC2 (NEEC)) only 5 proteins out of the 15 candidates were found to be differentially expressed in the verification phase (adj. p-value < 0.01). The verified biomarkers were CLD6, IF2B3, BCAM, PLD3 and MX1. A total of 4 out of the 45 quantified proteins presented high accuracy to individually discriminate between EC1 and EC2 cases with AUC values higher than 0.75: CLD6 (AUC= 0.88, CI95: 0.76-1.00), BCAM (AUC= 0.87, CI95: 0.76-0.97), IF2B3 (AUC=0.80, IC95: 0.68-0.93) and PLD3 (AUC= 0.79, IC95: 0.66-0.93). Table 2.2 below shows the list of selected biomarkers for EC prognosis in ELVs.

### 2.3. Verification of candidates by targeted proteomics in the whole fluid of UAs

In order to understand whether it would be feasible to transfer the use of these biomarkers to a sample that is easier to access and does not require the isolation of the extracellular vesicles, it was aimed to study the biomarkers identified in exosomes in the whole fluid fraction of UAs. For that, a total of 67 patients (cohort C) were selected and divided into three groups: EEC (n=22), NEEC (n=20) and CTRL (n=25).

For this part of the study, a total of 51 proteins were analyzed. Two unique tryptic peptides were selected per protein, from which a total of 75 endogenous peptides were finally monitored by scheduled-SRM. A total of 37 proteins were only detected and subsequently quantified for AUC estimation.

The results obtained in this verification study were evaluated in comparison to the one previously performed, in order to understand the feasibility to translate ELV's based biomarkers to the whole fluid of UAs. First, it was observed that the detectability of the ELVs biomarkers in the whole fluid of UAs was very limited compared to the detectability when ELVs were analyzed; out of the 51 biomarkers detected in ELVs, only 34 (66,7%) were detected in UAs.

In next Table 1.1 and Table 1.2, the list of significant biomarkers for EC diagnosis in whole fluid fraction of UAs, and for EC prognosis (comparison between EEC and NEEC) in said whole fluid fraction of UAs.

**Table 1.1. Biomarkers of diagnosis in uterine aspirates**

| **Biomarkers of diagnosis in uterine aspirates** | | | | | | | | | | |
|---|---|---|---|---|---|---|---|---|---|---|
| **Protein** | **log2FC** | **FC** | **SE** | **Tvalue** | **DF** | **pvalue** | **adj.pvalue** | **AUC** | **IC95%** | |
| sp\|P14780\|MMP9_HUMAN | -2.76 | 0.15 | 0.37 | -7.44 | 64 | 3.12E-10 | 1.15E-08 | .914 | .843 | .985 |
| sp\|000468\|AGRIN_HUMAN | -1.43 | 0.37 | 0.21 | -6.83 | 64 | 3.63E-09 | 6.72E-08 | .864 | .772 | .956 |
| sp\|Q14764\|MVP_HUMAN | -1.41 | 0.38 | 0.21 | -6.63 | 64 | 8.23E-09 | 1.01E-07 | .884 | 805 | .962 |
| sp\|Q9BV40\|VAMP8_HUMAN | -1.00 | 0.50 | 0.18 | -5.57 | 64 | 5.51E-07 | 5.10E-06 | .855 | .766 | .944 |
| sp\|P41252\|SYIC_HUMAN | -1.55 | 0.34 | 0.29 | -5.40 | 64 | 1.05E-06 | 7.76E-06 | .854 | .762 | 947 |
| sp\|P61006\|RAB8A_HUMAN | -1.11 | 0.46 | 0.21 | -5.23 | 64 | 1.96E-06 | 1.21E-05 | .836 | .737 | .936 |
| sp\|P20591\|MX1_HUMAN | -1.48 | 0.36 | 0.30 | -4.98 | 64 | 5.09E-06 | 2.69E-05 | .859 | .770 | .948 |
| sp\|Q14974\|IMB1_HUMAN | -1.68 | 0.31 | 0.35 | -4.81 | 64 | 9.39E-06 | 4.34E-05 | .849 | .756 | 941 |
| sp\|000299\|CLIC1_HUMAN | -1.38 | 0.38 | 0.29 | -4.78 | 64 | 1.07E-05 | 4.40E-05 | .832 | .736 | .929 |
| sp\|P62318\|SMD3_HUMAN | -1.11 | 0.46 | 0.24 | -4.72 | 64 | 1.31E-05 | 4.86E-05 | .792 | .683 | .902 |
| sp\|Q12905\|ILF2_HUMAN | -0.98 | 0.51 | 0.21 | -4.56 | 64 | 2.39E-05 | 8.03E-05 | .838 | .737 | .939 |
| sp\|P55072\|TERA_HUMAN | -0.77 | 0.59 | 0.17 | -4.41 | 64 | 4.09E-05 | 1.26E-04 | .789 | .682 | .895 |
| sp\|P62913\|RL11_HUMAN | -1.15 | 0.45 | 0.28 | -4.16 | 64 | 9.62E-05 | 2.74E-04 | .768 | .653 | .882 |
| sp\|P50895\|BCAM_HUMAN | -0.79 | 0.58 | 0.19 | -4.11 | 64 | 1.15E-04 | 3.04E-04 | .773 | 664 | .882 |
| sp\|P07355\|ANXA2_HUMAN | -1.15 | 0.45 | 0.28 | -4.04 | 64 | 1.48E-04 | 3.41E-04 | .759 | .642 | .876 |
| sp\|Q01650\|LAT1_HUMAN | -0.87 | 0.55 | 0.22 | -4.05 | 64 | 1.43E-04 | 3.41E-04 | .827 | .718 | 936 |
| sp\|Q9Y265\|RUVB1_HUMAN | -1.13 | 0.46 | 0.28 | -4.02 | 64 | 1.58E-04 | 3.45E-04 | .796 | .691 | .901 |
| sp\|Q9H299\|SH3L3_HUMAN | -0.83 | 0.56 | 0.21 | -3.95 | 64 | 2.00E-04 | 4.11E-04 | .774 | .663 | .885 |
| sp\|P40429\|RPL13A_HUMAN | -1.33 | 0.40 | 0.35 | -3.80 | 64 | 3.26E-04 | 6.35E-04 | 790 | .674 | 905 |
| sp\|P62249\|RS16_HUMAN | -1.24 | 0.42 | 0.33 | -3.69 | 64 | 4.59E-04 | 8.50E-04 | .693 | .562 | .825 |
| sp\|P08865\|RSSA_HUMAN | -1.01 | 0.50 | 0.28 | -3.59 | 64 | 6.40E-04 | 1.13E-03 | 782 | 670 | .894 |
| sp\|P30050\|RL12_HUMAN | -1.18 | 0.44 | 0.33 | -3.57 | 64 | 6.76E-04 | 1.14E-03 | .779 | 670 | .889 |
| sp\|P07237\|PDIA1_HUMAN | -0.68 | 0.62 | 0.19 | -3.55 | 64 | 7.25E-04 | 1.17E-03 | .795 | .689 | .901 |
| sp\|P47914\|RL29_HUMAN | -1.07 | 0.48 | 0.30 | -3.53 | 64 | 7.76E-04 | 1.20E-03 | .761 | .643 | .879 |
| sp\|P62937\|PPIA_HUMAN | -0.98 | 0.51 | 0.32 | -3.03 | 64 | 3.56E-03 | 5.26E-03 | .741 | .625 | .857 |
| sp\|O95994\|AGR2_HUMAN | -0.93 | 0.52 | 0.33 | -2.83 | 64 | 6.14E-03 | 8.74E-03 | .703 | 580 | .826 |
| sp\|P14618\|KPYM_HUMAN | -1.04 | 0.49 | 0.37 | -2.82 | 64 | 6.45E-03 | 8.84E-03 | .798 | .692 | 904 |

**Table 1.2. Biomarkers of prognosis in uterine aspirates**

| **Biomarkers of prognosis in uterine aspirates** | | | | | | | | | |
|---|---|---|---|---|---|---|---|---|---|
| **Protein** | **log2FC** | **SE** | **Tvalue** | **DF** | **pvalue** | **adj.pvalue** | **AUC** | **IC95%** | |
| sp\|P01833\|PIGR_HUMAN | -3.18 | 0.58 | -5.43 | 64.00 | 0 | 3.38E-05 | 8.80E-01 | .769 | .991 |
| sp\|O95994\|AGR2_HUMAN | -1.72 | 0.35 | -4.95 | 64.00 | 0 | 1.06E-04 | 8.30E-01 | .709 | .950 |
| sp\|P50895\|BCAM_HUMAN | 0.91 | 0.21 | 4.43 | 64.00 | 0 | 4.58E-04 | 1.66E-01 | .042 | .290 |
| sp\|O00592\|PODXL_HUMAN | -1.86 | 0.46 | -4.08 | 64.00 | 0 | 1.18E-03 | 8.43E-01 | .721 | .965 |
| sp\|P14780\|MMP9_HUMAN | -1.59 | 0.43 | -3.69 | 64.00 | 0 | 3.41E-03 | 8.02E-01 | .665 | .940 |
| sp\|P13987\|CD59_HUMAN | -0.94 | 0.28 | -3.32 | 64.00 | 0 | 9.17E-03 | 8.05E-01 | .660 | .949 |

**Table 2.1. Biomarkers of diagnosis in Exosome-containing fraction (ELV) isolated from UA:**

| **Entry Name** | **UniProt Number** | **FC** | **log2FC** | **adj.pvalue** | **AUC** | **IC95%** |
|---|---|---|---|---|---|---|
| AGRIN_HUMAN | O00468 | 4,46 | 2,16 | 1,25E-13 | **0,90** | 0,85 - 0,9$ |
| MVP_HUMAN | Q14764 | 7,50 | 2,91 | 3,01E-10 | **0,86** | 0,78 - 0,93 |
| TACD2_HUMAN | P09758 | 4,76 | 2,25 | 3,01E-10 | **0,87** | 0,81 - 0,94 |
| FAS_HUMAN | P49327 | 4,90 | 2,28 | 4,30E-10 | **0,85** | 0,78 - 0,92 |
| SYIC_HUMAN | P41252 | 9,74 | 3,28 | 1,20E-09 | **0,84** | 0,77 - 0,91 |
| VAMP8_HUMAN | Q9BV40 | 4,17 | 2,06 | 1,57E-09 | **0,84** | 0,76-0,92 |
| SORT_HUMAN | Q99523 | 3,10 | 1,63 | 1,57E-09 | **0,86** | 0,79-0,93 |
| LAT1_HUMAN | 001650 | 5,65 | 2,50 | 4,51E-09 | **0,84** | 0,76 - 0,91 |
| TERA_HUMAN | P55072 | 4,93 | 2,30 | 9,49E-09 | **0,82** | 0,74 - 0,90 |
| RUVB1_HUMAN | Q9Y265 | 4,76 | 2,25 | 9,49E-09 | **0,82** | 0,74 - 0,90 |
| RSSA_HUMAN | P08865 | 3,63 | 1,86 | 9,49E-09 | **0,82** | 0,74 - 0,90 |
| SMD3_HUMAN | P62398 | 4,38 | 2,13 | 1,09E-08 | **0,82** | 0,75 - 0,90 |
| ADA10_HUMAN | 014672 | 2,44 | 1,29 | 2,21E-08 | **0,80** | 0,71-0,89 |
| RPL13A_HUMAN | P40429 | 10,98 | 3,48 | 3,01E-08 | **0.80** | 0,72 - 0,88 |
| PGBM_HUMAN | P98160 | 3,95 | 1,98 | 3,05E-08 | n.q | n.q |
| RL11_HUMAN | P62913 | 8,32 | 3,06 | 3,53E-08 | **0.80** | 0,71 - 0.88 |
| IMB1_HUMAN | Q14974 | 3,27 | 1,71 | 3,53E-08 | **0,80** | 0,71 - 0,89 |
| AGR2_HUMAN | O95994 | 5,84 | 2,55 | 5,10E-08 | **0.79** | 0,69 - 0,88 |
| ITA3_HUMAN | P26006 | 2,26 | 1,18 | 5,10E-08 | **0,81** | 0,73 - 0,89 |
| RUXE_HUMAN | P62304 | 3,03 | 1,60 | 7,34E-08 | **0,78** | 0,70 - 0,87 |
| RL12_HUMAN | P30050 | 9,82 | 3,30 | 7,80E-08 | **0,79** | 0,71 - 0,88 |
| RS16_HUMAN | P62249 | 9,04 | 3,18 | 1,36E-07 | **0,79** | 0,71 - 0,87 |
| PSMD2_HUMAN | Q13200 | 5,09 | 2,35 | 3,02E-07 | **0,79** | 0,70 - 0,87 |
| MX1_HUMAN | P20591 | 3,94 | 1,98 | 3,77E-07 | **0,81** | 0,73 - 0,89 |
| VPS35_HUMAN | Q96QK1 | 2,00 | 1,00 | 7,86E-07 | **0,78** | 0,69 - 0,87 |
| ILF2_HUMAN | Q12905 | 3,2Q | 1,68 | 8,51E-07 | **0,79** | 0,70 - 0,88 |
| PDIA1_HUMAN | P07237 | 2,65 | 1,41 | 9,50E-07 | **0,77** | 0,68 - 0,86 |
| MMP9_HUMAN | P14780 | 1,97 | 0,98 | 7,67E-06 | **0,79** | 0,70 - 0,88 |
| ANXA4_HUMAN | P09525 | 3,58 | 1,84 | 1,20E-05 | 0,72 | 0,61 - 0,82 |
| RAB8A_HUMAN | P61006 | 3,18 | 1,67 | 1,20E-05 | 0,73 | 6,62 - 0,84 |
| SH3L3_HUMAN | Q9H299 | 2,32 | 1,22 | 1,28E-05 | 0,72 | 0,62 - 0,83 |
| RL29_HUMAN | P41914 | 7,00 | 2,81 | 1,55E-05 | 0,74 | 0,64 - 0,83 |
| PLD3_HUMAN | Q8IV08 | 1,74 | 0,80 | 1,82E-05 | **0,75** | 0,65 - 0,85 |
| PPIA_HUMAN | P62937 | 3,86 | 1,95 | 2.86E-05 | 0,71 | 0,60 - 0,82 |
| ANXA2_HUMAN | P07355 | 3,49 | 1,80 | 3.66E-05 | 0,70 | 0,59 - 0,81 |
| S10AC_HUMAN | P80511 | 7,75 | 2,95 | 5.40E-05 | n.q | n.q |
| CD14_HUMAN | P08571 | 3,89 | 1,96 | 6.48E-05 | n.q | n.q |
| SSRA_HUMAN | P43307 | 3,05 | 1,61 | 6,67E-05 | 0,72 | 0,61-0,82 |
| LAMP2_HUMAN | P13473 | 2,16 | 1,11 | 7.78E-05 | 0,72 | 0,62 - 0,83 |
| PODXL_HUMAN | 000592 | 2,86 | 1,52 | 2,61E-04 | 0,71 | 0,61 - 0,81 |
| CLD6_HUMAN | P56747 | 1,83 | 0,87 | 2,61E-04 | 0,67 | 0,57 - 0,77 |
| IF2B3_HUMAN | O00425 | 1,97 | 0,98 | 4,27E-04 | **0,77** | 0,68 - 0,86 |
| CD59_HUMAN | P13987 | 2,80 | 1,49 | 6,17E-04 | 0,64 | 0,52 - 0,76 |
| MLEC_HUMAN | 014165 | 2,05 | 1,04 | 2,55E-03 | 0,68 | 0,57 -0,78 |
| H10_HUMAN | P07305 | 1,65 | 0,72 | 6,93E-03 | n.q | n.q |
| CD166_HUMAN | Q13740 | 1,59 | 0,67 | 9,42E-03 | 0,66 | 0,55 - 0,77 |
| CD81_HUMAN | P60033 | 2,01 | 1,00 | 1,15E-02 | 0,63 | 0,51 - 0,75 |
| AR6P1_HUMAN | 015041 | 2,47 | 1,31 | 3,43E-02 | n.q | n.q |
| BCAM_HUMAN | P50895 | 1,24 | 0,31 | 3,78E-02 | 0,63 | 0,52 - 0,74 |
| VAC14_HUMAN | Q08AM6 | 1,61 | 0,68 | 5,29E-02 | n.q | n.q |
| ITB3_HUMAN | P05106 | 1,33 | 0,41 | 5,61E-02 | 0,61 | 0,50 - 0,73 |

**Table 2.2. Biomarkers for EC prognosis in ELVs.**

| **Entry Name** | **UniProt Number** | **FC** | **log2FC** | **adj.pvalue** | **AUC** | **IC95%** |
|---|---|---|---|---|---|---|
| CLD6_HUMAN | P56747 | 4,06 | 2,02 | 9,63E-13 | 0,88 | 0,76 - 1,00 |
| BCAM_HUMAN (*) | P50895 | 2,12 | 1,08 | 4.60E-08 | 0.87 | 0,76 - 0,97 |
| IF2B3_HUMAN | O00425 | 3,27 | 1,71 | 5,28E-06 | 0,80 | 0,68 - 0,93 |
| PLD3_HUMAN | Q8IV08 | 2,01 | 1,01 | 6.72E-05 | 0,79 | 0,66 - 0,93 |
| MX1_HUMAN | P20591 | 3,49 | 1,80 | 7,91E-04 | 0,74 | 0,61 - 0,87 |

### Citation List

- DeSouza LV, et al, "Endometrial cancer biomarker discovery and verification using differentially tagged clinical samples with multidimensional liquid chromatography and tandem mass spectrometry", Mol Cell Proteomics MCP- 2007, vol. no.6, pp.: 1170-8.
- Kemik P, et al. "Diagnostic and prognostic values of preoperative serum levels of YKL-40, HE-4 and DKK-3 in endometrial cancer", Gynecol Oncol- 2016; vol. no. 140, pp.:64-9.
- Martinez-Garcia E, et al. "Development of a sequential workflow based on LC-PRM for the verification of endometrial cancer protein biomarkers in uterine aspirate samples", Oncotarget -2016, vol. no. 7(33), pp.:53102-53114.
- Robin et al., "pROC: and open-source package for R and S+ to analyze and compare ROC curves", BMC Bioinformatics-2011, vol. no. 12:77.
- Mota et al., "Genetic analysis of uterine aspirates improves the diagnostic value and captures the intra-tumor heterogeneity of endometrial cancers", Modern Pathology-2016, vol. 30, no. 1, pp.: 134-145.
- Cabrera et al., "Targeted proteomics identifies proteomic signatures in liquid-biopsies of the endometrium to diagnose endometrial cancer and assist the prediction of the optimal surgical treatment", International Journal of Gynecological Cancer-2017, vol. 27, no. supplement 4, pp.: 340-340.
- Chakraborty et al., "An oncogenic role of Agrin in regulating focal adhesion integrity in hepatocellular carcinoma", Nature Communications-2015, vol. 6, page 6184.

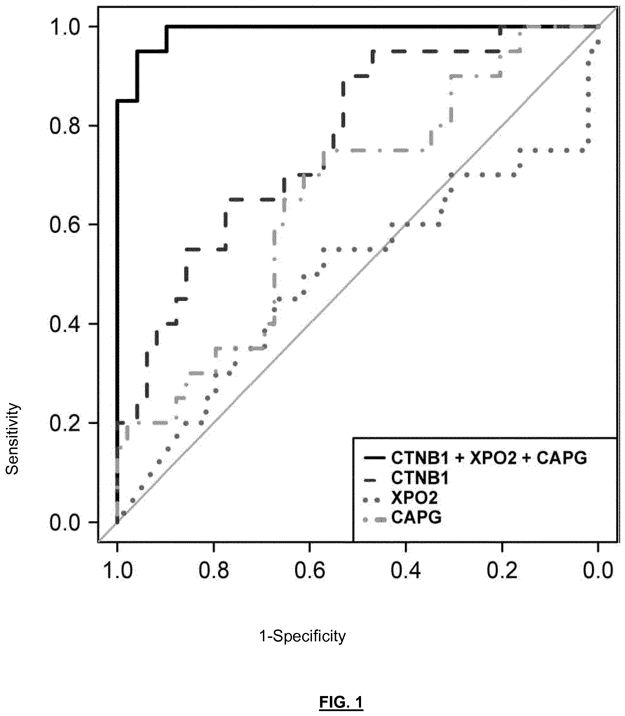

## Claims

1. A method of diagnosing endometrial cancer, the method comprising determining the level of expression of AGRIN in an uterine fluid sample from the female genital tract.

2. The method according to claim 1, wherein the uterine fluid sample is uterine aspirate fluid sample from the female genital tract.

3. The method according to any of claims 1-2, wherein the uterine fluid sample is exosome-containing fraction isolated from a uterine aspirate.

4. The method according to claim 3, comprising determining in an exosome-containing fraction isolated from a uterine aspirate the level of expression of at least one set of proteins selected from the group consisting of: AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; and AGRIN, BCAM.

5. The method according to claim 2, comprising determining in a uterine aspirate the level of expression of at least one set of proteins selected from the group consisting of: AGRIN, MMP9, PODXL; and AGRIN, MMP9.

6. The method according to any one of claims 1-3, further comprising determining the level of expression of one or more proteins selected from PERM, OSTP, CTNB1, CAYP1, XPO2, NGAL, SG2A1, CADH1, SPIT1, MMP9, NAMPT, LDHA, CASP3, PRDX1, MIF, K2C8, CAPG, MUC1, ANXA1, HSPB1, PIGR, CH10, CD44, CLIC1, TPIS, GSTP1, GTR1, ENOA, PDIA1, KPYM, ANXA2 and FABP5.

7. The method according to any one of claim 1-6, that comprises determining the level of expression of at least one set of proteins selected from the group consisting of: AGRIN, CD81, TERA; AGRIN, CD59, MVP; AGR2, AGRIN, CD81; AGRIN, CD166, MVP; AGRIN, CD81; AGRIN, CD166; AGRIN, CD59; and AGRIN, MMP9, in an exosome-containing fraction isolated from UA; and determining the level of expression of at least one set of proteins selected from the group consisting of: MMP9, PODXL, RAB8A; MMP9, PODXL, RSSA; AGRIN, MMP9, PODXL; MMP9, PODXL, VAMP8; MMP9, MX1; MMP9, RSSA; MMP9, MVP; MMP9, RAB8A; MMP9, VAMP8; BCAM, MMP9; MMP9, AGRIN in a uterine aspirate.

8. The method according to any of claims 1-7, wherein the level of expression is determined at the protein level.

9. The method according to any of claims 1-8, wherein the protein level is determined by an assay or technology selected from the group consisting of an immunoassay, a bioluminescence assay, a fluorescence assay, a chemiluminescence assay, electrochemistry assay, mass spectrometry, and combinations thereof.

10. The method according to any one of claims 1-9, which is carried out by means of a kit comprising a solid support and means for detecting the level of expression of AGRIN.

11. A kit comprising a solid support and means for detecting the level of expression of AGRIN and means for detecting the level of expression of the proteins of at least one set of proteins selected from the group consisting of AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; AGRIN, BCAM; AGRIN, MMP9, PODXL; AGRIN, MMP9; and AGRIN, MMP9, PIGR, wherein the means are antibodies or fragments thereof able to bind to the target protein(s).

12. The kit according to claim 11, wherein the antibodies or fragments thereof able to bind to the target protein(s) for detecting the level of expression of the proteins are antibodies or fragments thereof able to bind to the target protein(s) for carrying out an assay or technology selected from the group consisting of an immunoassay, a bioluminescence assay, a fluorescence assay, a chemiluminescence assay, electrochemistry assay, and combinations thereof.

13. The method according to any of claims 1-10, comprising:
a) determining, *in vitro,* the level of expression of AGRIN, in an uterine fluid sample from the female;
b) optionally determining, *in vitro,* the level of expression of AGRIN, in an exosome-containing fraction isolated from uterine fluid; and
c) comparing the level of step (a) and optionally that of step (b) with a reference control level, wherein if the level determined in step (a) and optionally that of step (b) is higher than the reference control level, it is indicative that the subject is suspicious of suffering endometrial carcinoma.

14. A method of deciding or recommending whether to initiate a medical regimen of a subject suspicious of suffering endometrial carcinoma, which method comprises the steps of:
a) determining, *in vitro,* the level of expression of AGRIN in an uterine fluid sample from the female;
b) optionally determining, *in vitro,* the level of expression of AGRIN in an exosome-containing fraction isolated from uterine fluid; and
c) comparing the level of step (a) and optionally that of step (b) with a reference control level, wherein if the level determined in step (a) and optionally that of step (b) is higher than the reference control level, it is indicative that the subject is suspicious of suffering endometrial carcinoma.
wherein:
i) if the subject is diagnosed of suffering from endometrial carcinoma, or of being suspicious of suffering from endometrial carcinoma, then the initiation of the medical regimen is recommended, and
ii) if the patient is diagnosed of not suffering from endometrial carcinoma, the follow-up is performed optionally in consideration of the result of an examination of the patient by a physician.

15. A method according to any of claims 1-10, in which after the determination of the level of expression of AGRIN, and the proteins of the sets of proteins for the diagnosis of EC, the following computer-implemented steps are carried out: said level(s) are given a value and/or a score, and optionally are computed in a mathematical formula to obtain a computed value; wherein in function of the said level(s), score(s) and or computed value(s), a decision is taken between the options of suffering or not from EC.

16. Use of a kit comprising a solid support and means for detecting the level of expression of AGRIN and, optionally, means for detecting the level of expression of the proteins of at least one set of proteins selected from the group consisting of AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; AGRIN, BCAM; AGRIN, MMP9, PODXL; AGRIN, MMP9; and AGRIN, MMP9, PIGR, for the diagnosis of endometrial cancer, wherein the means are antibodies or fragments thereof able to bind to the target protein(s).

17. The use of the kit according to claim 16, wherein the antibodies or fragments thereof able to bind to the target protein(s) for detecting the level of expression of the proteins are antibodies or fragments thereof able to bind to the target protein(s) for carrying out an assay or technology selected from the group consisting of an immunoassay, a bioluminescence assay, a fluorescence assay, a chemiluminescence assay, electrochemistry assay, and combinations thereof.

## Patentansprüche

1. Verfahren zur Diagnose von Gebärmutterkrebs, wobei das Verfahren das Bestimmen des Expressionsniveaus von AGRIN in einer Uterusflüssigkeitsprobe aus dem weiblichen Genitaltrakt umfasst.

2. Verfahren nach Anspruch 1, wobei die Uterusflüssigkeitsprobe eine Uterusaspirat-Flüssigkeitsprobe aus dem weiblichen Genitaltrakt ist.

3. Verfahren nach einem der Ansprüche 1-2, wobei die Uterusflüssigkeitsprobe eine aus einem Uterusaspirat isolierten Exosom-enthaltende Fraktion ist.

4. Verfahren nach Anspruch 3, umfassend das Bestimmen in einer Exosom-enthaltenden Fraktion, welche aus einem Uterusaspirat isoliert wurde, des Expressionsniveaus von mindestens einem Satz von Proteinen ausgewählt aus der Gruppe bestehend aus: AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; und AGRIN, BCAM.

5. Verfahren nach Anspruch 2, umfassend das Bestimmen in einem Uterusaspirat des Expressionsniveaus von mindestens einem Satz von Proteinen ausgewählt aus der Gruppe bestehend aus: AGRIN, MMP9, PODXL; und AGRIN, MMP9.

6. Verfahren nach einem der Ansprüche 1-3, zusätzlich umfassend das Bestimmen des Expressionsniveaus einer oder mehrerer Proteinen ausgewählt aus PERM, OSTP, CTNB1, CAYP1, XPO2, NGAL, SG2A1, CADH1, SPIT1, MMP9, NAMPT, LDHA, CASP3, PRDX1, MIF, K2C8, CAPG, MUC1, ANXA1, HSPB1, PIGR, CH10, CD44, CLIC1, TPIS, GSTP1, GTR1, ENOA, PDIA1, KPYM, ANXA2 und FABP5.

7. Verfahren nach einem der Ansprüche 1-6, welches das Bestimmen des Expressionsniveaus von mindestens einem Satz von Proteinen ausgewählt aus der Gruppe bestehend aus: AGRIN, CD81, TERA; AGRIN, CD59, MVP; AGR2, AGRIN, CD81; AGRIN, CD166, MVP; AGRIN, CD81; AGRIN, CD166; AGRIN, CD59; und AGRIN, MMP9, in einer Exosom-enthaltenden Fraktion, welche aus UA isoliert wurde; und das Bestimmen des Expressionsniveaus von mindestens einem Satz von Proteinen ausgewählt aus der Gruppe bestehend aus: MMP9, PODXL, RAB8A; MMP9, PODXL, RSSA; AGRIN, MMP9, PODXL; MMP9, PODXL, VAMP8; MMP9, MX1; MMP9, RSSA; MMP9, MVP; MMP9, RAB8A; MMP9, VAMP8; BCAM, MMP9; MMP9, AGRIN in einem Uterusaspirat, umfasst.

8. Verfahren nach einem der Ansprüche 1-7, wobei das Expressionsniveau am Proteinniveau bestimmt wird.

9. Verfahren nach einem der Ansprüche 1-8, wobei das Proteinniveau mittels eines Tests oder einer Technologie ausgewählt aus der Gruppe bestehend aus einem Immuntest, einem Biolumineszenztest, einem Fluoreszenztest, einem Chemolumineszenztest, einem Elektrochemietest, Massenspektrometrie und Kombinationen derselben bestimmt wird.

10. Verfahren nach einem der Ansprüche 1-9, welches mittels eines Kits umfassend einen festen Träger und Mittel zur Erfassung des Expressionsniveaus von AGRIN durchgeführt wird.

11. Kit umfassend einen festen Träger und Mittel zur Erfassung des Expressionsniveaus von AGRIN und Mittel zur Erfassung des Expressionsniveau der Proteine von mindestens einem Satz von Proteinen ausgewählt aus der Gruppe bestehend aus AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; AGRIN, BCAM; AGRIN, MMP9, PODXL; AGRIN, MMP9; und AGRIN, MMP9, PIGR, wobei die Mittel Antikörper oder Fragmente derselben sind, welche in der Lage sind, sich an das Zielprotein/die Zielproteine zu binden.

12. Kit nach Anspruch 11, wobei die Antikörper oder Fragmente derselben, welche in der Lage sind, sich an das Zielprotein/die Zielproteine zu binden, zur Erfassung des Expressionsniveaus der Proteine Antikörper oder Fragmente derselben sind, welche in der Lage sind, sich an das Zielprotein/die Zielproteine zu binden, um einen Test oder eine Technologie ausgewählt aus der Gruppe bestehend aus einem Immuntest, einem Biolumineszenztest, einem Fluoreszenztest, einem Chemolumineszenztest, einem Elektrochemietest, Massenspektrometrie, und Kombinationen derselben durchzuführen.

13. Verfahren nach einem der Ansprüche 1-10, umfassend:
a) das Bestimmen, *in vitro,* des Expressionsniveaus von AGRIN in einer Uterusflüssigkeitsprobe der Frau;
b) das wahlweise Bestimmen, *in vitro,* des Expressionsniveaus von AGRIN in einer Exosom-enthaltenden Fraktion, welche aus Uterusflüssigkeit isoliert wurde; und
c) das Vergleichen des Niveaus aus Schritt (a) und wahlweise des Niveaus aus Schritt (b) mit einem Referenzsteuerniveau, wobei, wenn das in Schritt (a) bestimmte Niveau und wahlweise das Niveau aus Schritt (b) höher als das Referenzsteuerniveau ist, es darauf schließen lässt, dass es verdacht wird, dass das Individuum unter Endometriumkarzinom leidet.

14. Verfahren zum Entscheiden oder Empfehlen, ob eine medizinische Kur eines Individuums, von welche verdacht wird, dass es unter Endometriumkarzinom leidet, angefangen werden soll, welches Verfahren die folgenden Schritte umfasst:
a) das Bestimmen, *in vitro,* des Expressionsniveaus von AGRIN in einer Uterusflüssigkeitsprobe der Frau;
b) das wahlweise Bestimmen, *in vitro,* des Expressionsniveaus von AGRIN in einer Exosom-enthaltenden Fraktion, welche aus Uterusflüssigkeit isoliert wurde; und
c) das Vergleichen des Niveaus aus Schritt (a) und wahlweise des Niveaus aus Schritt (b) mit einem Referenzsteuerniveau, wobei, wenn das in Schritt (a) bestimmte Niveau und wahlweise das Niveau aus Schritt (b) höher als das Referenzsteuerniveau ist, es darauf schließen lässt, dass es verdacht wird, dass das Individuum unter Endometriumkarzinom leidet,
wobei:
i) wenn es diagnostiziert wird, dass das Individuum unter Endometriumkarzinom leidet, oder es verdacht wird, dass es unter Endometriumkarzinom leidet, dann der Anfang der medizinische Kur empfohlen wird, und
ii) wenn es diagnostiziert wird, dass die Patientin nicht unter Endometriumkarzinom leidet, die Überwachung wahlweise unter Berücksichtigung des Ergebnisses einer Untersuchung der Patientin vonseiten eines Arztes durchgeführt wird.

15. Verfahren nach einem der Ansprüche 1-10, in welchem, nach der Bestimmung des Expressionsniveaus von AGRIN, und der Proteine des Satzes von Proteinen zur Diagnose von EC, die folgenden computerimplementierten Schritte durchgeführt werden: das genannte Niveau/die genannten Niveaus bekommen einen Wert und/oder eine Punktzahl, und werden wahlweise in einer mathematischen Formel berechnet, um einen berechneten Wert zu erhalten; wobei, in Abhängigkeit des genannten Niveaus/der genannten Niveaus, der genannten Punktzahl(en) und/oder des berechneten Wertes/der berechneten Werte, eine Entscheidung zwischen den Optionen unter EC zu leiden oder nicht unter EC zu leiden getroffen wird.

16. Verwendung eines Kits umfassend einen festen Träger und Mittel zur Erfassung des Expressionsniveaus von AGRIN und, wahlweise, Mittel zur Erfassung des Expressionsniveaus der Proteine von mindestens einem Satz von Proteinen ausgewählt aus der Gruppe bestehend aus AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9; AGRIN, BCAM; AGRIN, MMP9, PODXL; AGRIN, MMP9; und AGRIN, MMP9, PIGR, zur Diagnose von Gebärmutterkrebs, wobei die Mittel Antikörper oder Fragmente derselben sind, welche in der Lage sind, sich an das Zielprotein/die Zielproteine zu binden.

17. Verwendung des Kits nach Anspruch 16, wobei die Antikörper oder Fragmente derselben, welche in der Lage sind, sich an das Zielprotein/die Zielproteine zu binden, zur Erfassung des Expressionsniveaus der Proteine Antikörper oder Fragmente derselben sind, welche in der Lage sind, sich an das Zielprotein/die Zielproteine zu binden, um einen Test oder eine Technologie ausgewählt aus der Gruppe bestehend aus einem Immuntest, einem Biolumineszenztest, einem Fluoreszenztest, einem Chemolumineszenztest, einem Elektrochemietest, Massenspektrometrie, und Kombinationen derselben durchzuführen.

## Revendications

1. Procédé de diagnostic de cancer endométrial, le procédé comprenant déterminer le niveau d'expression d'AGRIN dans un échantillon de fluide utérin à partir du tractus génital féminin.

2. Procédé selon la revendication 1, dans lequel l'échantillon de fluide utérin est un échantillon de fluide d'aspiration utérine à partir du tractus génital féminin.

3. Procédé selon l'une quelconque des revendications 1-2, dans lequel l'échantillon de fluide utérin est une fraction contenant exosome isolée à partir d'une aspiration utérine.

4. Procédé selon la revendication 3, comprenant déterminer dans une fraction contenant exosome isolée à partir d'une aspiration utérine le niveau d'expression d'au moins un ensemble de protéines sélectionné parmi le groupe consistant en : AGRIN, CD81, TERA ; AGRIN, CD59, MVP ; AGR2, AGRIN, CD81 ; AGRIN, CD166, MVP ; AGRIN, CD81 ; AGRIN, CD166 ; AGRIN, CD59 ; AGRIN, MMP9 ; et AGRIN, BCAM.

5. Procédé selon la revendication 2, comprenant déterminer dans une aspiration utérine le niveau d'expression d'au moins un ensemble de protéines sélectionné parmi le groupe consistant en : AGRIN, MMP9, PODXL ; et AGRIN, MMP9.

6. Procédé selon l'une quelconque des revendications 1-3, comprenant en outre déterminer le niveau d'expression d'une ou plusieurs protéines sélectionnées de PERM, OSTP, CTNB1, CAYP1, XPO2, NGAL, SG2A1, CADH1, SPIT1, MMP9, NAMPT, LDHA, CASP3, PRDX1, MIF, K2C8, CAPG, MUC1, ANXA1, HSPB1, PIGR, CH10, CD44, CLIC1, TPIS, GSTP1, GTR1, ENOA, PDIA1, KPYM, ANXA2 et FABP5.

7. Procédé selon l'une quelconque des revendications 1-6, qui comprend déterminer le niveau d'expression d'au moins un ensemble de protéines sélectionné parmi le groupe consistant en : AGRIN, CD81, TERA; AGRIN, CD59, MVP; AGR2, AGRIN, CD81 ; AGRIN, CD166, MVP; AGRIN, CD81 ; AGRIN, CD166 ; AGRIN, CD59 ; et AGRIN, MMP9, dans une fraction contenant exosome isolée à partir d'une AU ; et déterminer le niveau d'expression d'au moins un ensemble de protéines sélectionné parmi le groupe consistant en : MMP9, PODXL, RAB8A ; MMP9, PODXL, RSSA ; AGRIN, MMP9, PODXL; MMP9, PODXL, VAMP8 ; MMP9, MX1 ; MMP9, RSSA; MMP9, MVP; MMP9, RAB8A ; MMP9, VAMP8 ; BCAM, MMP9 ; MMP9, AGRIN dans une aspiration utérine.

8. Procédé selon l'une quelconque des revendications 1-7, dans lequel le niveau d'expression est déterminé au niveau de protéines.

9. Procédé selon l'une quelconque des revendications 1-8, dans lequel le niveau de protéines est déterminé par un test ou une technologie sélectionné(e) parmi le groupe consistant en un test immunologique, un test de bioluminescence, un test de fluorescence, un test de chimiluminescence, un test d'électrochimie, spectrométrie de masse et des combinaisons de ceux-ci.

10. Procédé selon l'une quelconque des revendications 1-9, qui est mis en oeuvre au moyen d'un kit comprenant un support solide et des moyens pour détecter le niveau d'expression d'AGRIN.

11. Kit comprenant un support solide et des moyens de détection du niveau d'expression d'AGRIN et des moyens pour détecter le niveau d'expression des protéines d'au moins un ensemble de protéines sélectionné parmi le groupe consistant en AGRIN, CD81, TERA; AGRIN, CD59, MVP; AGR2, AGRIN, CD81 ; AGRIN, CD166, MVP; AGRIN, CD81 ; AGRIN, CD166 ; AGRIN, CD59 ; AGRIN, MMP9 ; AGRIN, BCAM ; AGRIN, MMP9, PODXL ; AGRIN, MMP9 ; et AGRIN, MMP9, PIGR, dans lequel les moyens sont des anticorps ou des fragments de ceux-ci capables de se lier à la/aux protéine(s) cible(s).

12. Kit selon la revendication 11, dans lequel les anticorps ou fragments de ceux-ci capables de se lier à la/aux protéine(s) cible(s) pour détecter le niveau d'expression des protéines sont des anticorps ou des fragments de ceux-ci capables de se lier à la/aux protéine(s) cible(s) pour mettre en oeuvre un test ou une technologie sélectionné(e) parmi le groupe consistant en un test immunologique, un test de bioluminescence, un test de fluorescence, un test de chimiluminescence, un test d'électrochimie et des combinaisons de ceux-ci.

13. Procédé selon l'une quelconque des revendications 1-10, comprenant :
a) déterminer, *in vitro,* le niveau d'expression d'AGRIN dans un échantillon de fluide utérin de la femme ;
b) déterminer optionnellement, *in vitro,* le niveau d'expression d'AGRIN dans une fraction contenant exosome isolée à partir du fluide utérin ; et
c) comparer le niveau de l'étape (a) et optionnellement celui de l'étape (b) avec un niveau de contrôle de référence, dans lequel, si le niveau déterminé dans l'étape (a) et optionnellement celui de l'étape (b) est plus élevé que le niveau de contrôle de référence, cela est révélateur que le sujet est suspect de souffrir d'un carcinome endométrial.

14. Procédé pour décider ou recommander si initier un régime médical d'un sujet suspect de souffrir d'un carcinome endométrial, dont le procédé comprend les étapes suivantes de :
a) déterminer, *in vitro,* le niveau d'expression d'AGRIN dans un échantillon de fluide utérin de la femme ;
b) déterminer optionnellement, *in vitro,* le niveau d'expression d'AGRIN dans une fraction contenant exosome isolée à partir du fluide utérin ; et
c) comparer le niveau de l'étape (a) et optionnellement celui de l'étape (b) avec un niveau de contrôle de référence, dans lequel, si le niveau déterminé dans l'étape (a) et optionnellement celui de l'étape (b) est plus élevé que le niveau de contrôle de référence, cela est révélateur que le sujet est suspect de souffrir d'un carcinome endométrial ;
dans lequel :
i) si le sujet est diagnostiqué souffrant d'un carcinome endométrial, ou étant suspect de souffrir de carcinome endométrial, l'initiation d'un régime médical est alors recommandée, et
ii) si la patiente est diagnostiquée non souffrant d'un carcinome endométrial, le suivi est réalisé optionnellement compte tenu du résultat d'un examen de la patiente par un médecin.

15. Procédé selon l'une quelconque des revendications 1-10, dans lequel, après la détermination du niveau d'expression d'AGRIN, et des protéines des ensembles de protéines pour le diagnostic de CE, les étapes suivantes implémentées par ordinateur sont mis en oeuvre : une valeur et/ou un score est donné audit/auxdits niveau(x), et ils sont optionnellement calculés à partir d'une formule mathématique pour obtenir une valeur calculée ; dans lequel en fonction dudit/desdits niveau(x), score(s) et/ou valeur(s) calculée(s), une décision est prise entre les options de souffrir ou non de CE.

16. Utilisation d'un kit comprenant un support solide et des moyens pour détecter le niveau d'expression d'AGRIN et, optionnellement, des moyens pour détecter le niveau d'expression des protéines d'au moins un ensemble de protéines sélectionné parmi le groupe consistant en AGRIN, CD81, TERA; AGRIN, CD59, MVP ; AGR2, AGRIN, CD81 ; AGRIN, CD166, MVP ; AGRIN, CD81 ; AGRIN, CD166 ; AGRIN, CD59 ; AGRIN, MMP9; AGRIN, BCAM ; AGRIN, MMP9, PODXL ; AGRIN, MMP9 ; et AGRIN, MMP9, PIGR, pour le diagnostic du cancer endométrial, dans lequel les moyens sont des anticorps ou des fragments de ceux-ci capables de se lier à la/aux protéine(s) cible(s).

17. Utilisation du kit selon la revendication 16, dans lequel les anticorps ou des fragments de ceux-ci capables de se lier à la/aux protéine(s) cible(s) pour détecter le niveau d'expression des protéines sont des anticorps ou des fragments de ceux-ci capables de se lier à la/aux protéine(s) cible(s) pour mettre en oeuvre un test ou une technologie sélectionné(e) parmi le groupe consistant en un test immunologique, un test de bioluminescence, un test de fluorescence, un test de chimiluminescence, un test d'électrochimie et des combinaisons de ceux-ci
